(19) Europäisches Patentamt / European Patent Office / Office européen des brevets

(11) **EP 4 703 468 A1**

(12) **EUROPEAN PATENT APPLICATION**

(43) Date of publication:
04.03.2026 Bulletin 2026/10

(21) Application number: 24197402.1

(22) Date of filing: **29.08.2024**

(51) International Patent Classification (IPC):
*C12N 7/00* (2006.01)    *C12N 15/86* (2006.01)

(52) Cooperative Patent Classification (CPC):
**C12N 7/00; C12N 15/86;** C12N 2750/14122;
C12N 2750/14143

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(71) Applicant: **Sartorius Albumedix Limited**
**Nottingham NG2 3ED (GB)**

(72) Inventors:
• **RAWSTHORNE,, Helen**
Nottingham, NG2 3ED (GB)
• **MILLWATER,, Sophie Jade**
Nottingham, NG2 3ED (GB)

(74) Representative: **Potter Clarkson**
**Chapel Quarter**
**Mount Street**
**Nottingham NG1 6HQ (GB)**

(54) **METHODS AND USES**

(57)    The invention provides a method of preserving the activity of an adeno-associated virus (AAV) in the presence of an anti-AAV neutralising antibody, the method comprising contacting the AAV with albumin. Also provided is a method of improving the efficacy of an adeno-associated virus (AAV) based therapy, the method comprising contacting an AAV with albumin prior to administering the AAV to a subject.

# Figure 1

## Description

Gene therapy aims to cure a disease using genetic modification (Earley *et al*, 2023). Gene therapies have many ways of getting a corrective gene into a cell, and a widely known mechanism is the use of a viral vector such as a Retrovirus, an Adenovirus, or an Adeno Associated Virus (AAV).

AAV is a non-enveloped, single stranded DNA virus with an icosahedral capsid (Wang *et al.,* 2024). It is part of the Parvoviridae family, known as a 'Dependovirus' meaning it is unable to replicate or transcribe its genome without the assistance of a helper virus, such as Adenovirus or Herpes Simplex Virus (HSV) (Matsushita *et al.,* 1998). Due to its dependency on helper viruses, AAV is considered a safer alternative to other viral vectors as it is incapable of replicating and manufacturing new virus particles independently. Despite this, it has been known to latently integrate into the host cell genome (Laughlin *et al.,* 1986), which is a feature that can be utilised to provide long term gene expression in viral vector gene therapy. A key feature of AAV is that there are many viral serotypes which have specific tropisms for different tissues, for example, AAV8 has a tropism for liver cells and AAV3 has a tropism for cochlear inner hair cells (Issa *et al.,* 2023). This tropism means AAV is effective in treating monogenic rare diseases through gene therapy. An example of this is AAV9, which can cross the blood brain barrier, meaning it is used in a US Food and Drug Administration approved treatment of Spinal Muscular Atrophy, commercially known as ZOLGENSMA (Sarkar *et al.,* 2024).

The presence of neutralising antibodies is of concern in gene therapy because it means AAV virus particles will be neutralised before they are able to infect and deliver a corrective gene to a patient's cells. Even a low concentration of neutralising antibodies can reduce transduction efficiency levels (Gross *et al.,* 2022). In many clinical trials for AAV gene therapies, there is an exclusion criterion for patients who have neutralising antibodies against the AAV serotype of the therapy, meaning these patients are unable to be treated with AAV gene therapy. A neutralising antibody concentration as low as 1:40 was the cut off for eligibility to be included in a recent clinical trial treating GM1 gangliosidosis (Mendell *et al.,* 2022). Despite AAV being the viral vector gene therapy of choice (Earley *et al.,* 2023), a key drawback is that AAV is ubiquitous so many individuals likely have already been exposed to an AAV serotype. In a screening of 888 human serum samples, Calcedo & Wilson (2013) found many samples were seropositive for AAV2 as well as AAV1, AAV7 and AAV8. This demonstrates the prevalence of anti-AAV neutralising antibodies that will block the activity of AAV upon re-exposure.

Current methods for overcoming the barrier of neutralising antibodies involve subjecting a patient to immunosuppression or plasmapheresis. Immunosuppression involves the administration of drugs that attenuate a patient's immune system, preventing a patient's immune system working effectively and generating neutralising antibodies. Alternatively, plasmapheresis involves a patient's blood being washed through a column that binds and removes neutralising antibodies before being returned to them (Earley *et al.,* 2023). However, these procedures are laborious and intensive for a patient to undergo and will not necessarily deplete neutralising antibodies to an acceptable level that would avoid exclusion from a clinical trial. Neutralising antibodies reduce the transduction efficiency of AAV in gene therapies to a level much lower than is effective for delivering a corrective gene, meaning that more AAV would be required, providing a greater cost but no guarantee that this increase will combat the neutralisation. Even if a higher viral load could avoid a neutralising antibody response, a key drawback of immunosuppression and plasmapheresis remains that the process of removing the defensive response to the AAV gene therapy leaves a patient's immune system vulnerable to other infections (Earley *et al.,* 2023).

Albumin is a heart shaped protein with a molecular weight of 66.5 kDa (Sand *et al.,* 2014). Making up 60% of the total protein content in serum, albumin is fundamental in many cellular processes. It is known to work well as an antioxidant due to the presence of a free thiol group on the Cysteine 34 residue (Francis, 2010). This becomes reduced in the presence of free radicals, allowing the albumin to buffer cells during oxidative stress. Albumin is a key component in cell culture media and is found within Fetal Bovine Serum (FBS). However, few cell and gene therapy manufacturers use FBS and instead opt for xeno-component free alternatives to grow their cells, such as, Human Serum Albumin (HSA). Despite this there can be great batch to batch variation and issues in sourcing HSA, so, many manufacturers are switching to using recombinant albumin, such as Recombumin®, a yeast derived albumin.

The inventors have now found that incubating AAV in the presence of albumin provides a protective effect against neutralising antibodies. Specifically, the inventors conducted experiments testing the transduction efficiency of AAV preincubated with and without albumin in cells that had been exposed to a serial dilution of neutralising antibody and found that the transduction was not impaired by the neutralising antibodies if the AAV had been preincubated with albumin. This surprising effect was seen in multiple AAV serotypes and cell lines and has numerous clinical implications. The use of albumin may enable an individual to undergo an AAV treatment, or clinical trial, by reducing the maximum neutralising antibody exclusion criteria without weakening a patient's immune system or by reducing the dose of the AAV required.

Wang et al. (2017), found that if Adeno Associated Virus (AAV) is pre-incubated with HSA, the transduction efficiency of the virus is increased. The improvement in transduction efficiency found by Wang et al. (2017) was suggested to work by direct interaction between albumin and the AAV capsid, which could help facilitate its entry into cells as takes place with nano particles (Mariam *et al.,* 2016). However, the authors of Wang *et al.* suggested that the interaction of albumin with AAV did not interfere with neutralising antibody activity.

Accordingly, a first aspect of the invention provides a method of preserving the activity of an adeno-associated virus (AAV) in the presence of an anti-AAV neutralising antibody, the method comprising contacting the AAV with albumin.

Similarly, the invention provides the use of albumin to preserve the activity of an AAV in the presence of an anti-AAV neutralising antibody, wherein the use involves contacting the AAV with albumin.

By 'preserving the activity of an AAV in the presence of an anti-AAV neutralising antibody' we include the meaning that any one or more activities of the AAV are retained in the presence of an anti-AAV neutralising antibody. Thus, the activity of the AAV can be said to be maintained or safeguarded in the presence of an anti-AAV neutralising antibody. For example, by contacting the AAV with albumin, it is expected that the level of one or more activities of the AAV in the presence of an anti-AAV neutralising antibody will be greater than the respective level of the one or more activities of the AAV in the presence of an anti-AAV neutralising antibody when the AAV is not contacted with albumin.

Typically, the level of the activity of the AAV in the presence of an anti-AAV neutralising antibody when the AAV is contacted with albumin is at least 1% greater than the activity of the AAV in the presence of an anti-AAV neutralising antibody when the AAV is not contacted with albumin, for example at least 2%, 3%, 4%, 5%, 6%, 7%, 8%, 9% or 10% greater, such as at least 15%, 20%, 25%, 30%, 35%, 40%, 45% or 50% greater. It is preferred if the activity is at least 5%, 6%, 7%, 8%, 9%, 10%, 11%, 12%, 13%, 14%, 15%, 16%, 17%, 18%, 19% or 20% greater to at least 10%, 11%, 12%, 13%, 14%, 15%, 16%, 17%, 18%, 19%, 20%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49% or 50% greater. It is particularly preferred if the level of the activity of the AAV in the presence of an anti-AAV neutralising antibody when the AAV is contacted with albumin is from 5% to 50% greater, for example from 10% to 40% greater or from 20% to 30% greater, such as about 20% greater.

By activity of an AAV, we include any activity of the virus, for example one that may be affected or impaired by the presence of an anti-AAV neutralising antibody. Examples of such activities include the ability of the AAV to bind to a binding partner such as a cell surface receptor; the ability of the AAV to be endocytosed into a cell; the ability of the AAV to be trafficked through the endosome system; the ability of the AAV to be taken up into the nucleus; and the ability of the AAV to be uncoated. Also included is the ability of the AAV to integrate into a host genome. It will be appreciated that many of these examples of AAV activities are steps of virus transduction or infection, and so also included in "activity of an AAV" is the ability of the AAV to transduce or infect a cell.

Thus, in a preferred embodiment, the invention provides a method of preserving the ability of an AAV to transduce or infect a cell in the presence of an anti-AAV neutralising antibody, the method comprising contacting the AAV with albumin. Similarly, in a preferred embodiment, the invention provides the use of albumin to preserve the ability of an AAV to transduce or infect a cell in the presence of an anti-AAV neutralising antibody, wherein the use involves contacting the AAV with albumin.

In a particularly preferred embodiment, the activity of an AAV is the ability to transduce a cell. Typically, the level of transduction of the AAV into the cell in the presence of an anti-AAV neutralising antibody when the AAV is contacted with albumin is at least 1% higher than the level of transduction of the AAV into the cell in the presence of an anti-AAV neutralising antibody when the AAV is not contacted with albumin, for example at least 2%, 3%, 4%, 5%, 6%, 7%, 8%, 9% or 10% higher, such as at least 15%, 20%, 25%, 30%, 35%, 40%, 45% or 50% higher. It is preferred if the level of transduction is at least 5%, 6%, 7%, 8%, 9%, 10%, 11%, 12%, 13%, 14%, 15%, 16%, 17%, 18%, 19% or 20% higher to at least 10%, 11%, 12%, 13%, 14%, 15%, 16%, 17%, 18%, 19%, 20%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49% or 50% higher. It is particularly preferred if the level of transduction of the AAV into the cell in the presence of an anti-AAV neutralising antibody when the AAV contacted with albumin is from 5% to 50% higher, for example from 10% to 40% higher or from 20% to 30% higher, such as about 20% higher than the level of transduction of the AAV into the cell in the presence of an anti-AAV neutralising antibody when the AAV is not contacted with albumin.

Methods of assessing activities of an AAV are known in the art and the skilled person would be able to identify an appropriate method for the particular activity in question. Examples of such methods are described below and in the Examples.

When the activity of the AAV is the ability of AAV to transduce or infect a cell, conveniently, the virus contains a detectable nucleic acid in its genome, which nucleic acid can be detected inside the cell once the virus has transduced the cell. The detectable nucleic acid could be a nucleic acid that is detectable directly. For example, the nucleic acid may be detected following hybridisation with a probe that comprises a detectable moiety (e.g. a fluorophore or radiolabel). The nucleic acid may be detected indirectly, for example following expression of a protein encoded by the nucleic acid which protein can be detected, for example by virtue of assessing its expression or activity. It will be appreciated that the genome of the virus may be modified to include the detectable nucleic acid which can serve as a so-called "reporter gene" to measure the level of transduction. In this work, the reporter gene green fluorescent protein (GFP) was utilised as the transgene, and GFP expression in a cell was monitored to determine the levels of transduction enhancement. Other suitable reporter genes are known in the art and include yellow fluorescent protein, lacZ, alkaline phosphatase, and firefly luciferase.

In yet a further example, the AAV may contain a reporter or marker such as a fluorescent marker or antibiotic resistance marker. The number of fluorescent cells or antibiotic resistant cells in the population can then be quantified. For example, flow cytometry with a fluorescent protein marker (*e.g.*, fluorescence-activated cell sorting (FACS)) or antibiotic selection can be used to determine the percentage of infected cells. In a particular example, AAV particles may comprise a gene encoding a reporter or marker (e.g., fluorescent protein) under the control of a promoter, such that when the AAV is transduced into a cell, the gene is expressed, and the reporter or marker (e.g., a fluorescent protein) is produced. A plate reader can be used to measure bioluminescence of a luminescent marker. A live cell imager (such as an Incucyte® live-cell analysis system, Sartorius) can be used to measure a fluorescent protein.

Other examples of methods to assess the ability of AAV to transduce or infect a cell include a fluorescence-based method, bioluminescence (e.g. using the luciferase reporter gene), an enzyme-based method (e.g. using a beta galactosidase (LacZ) reporter gene), DNA staining, polymerase chain reaction (PCR) (e.g. reverse transcription PCR), enzyme-linked immunosorbent assay (ELISA) or western blotting. Quantitative PCR may be used, which allows for quantification of viral genome copies in transduced cells. By comparing viral DNA levels to a reference gene, transduction efficiency can be estimated. Immunofluorescence staining may be used which makes use of antibodies specific to AAV capsid proteins (such as VP1, VP2, or VP3) to detect transduced cells and fluorescent microscopy to reveal the presence of AAV particles in a cell population. Vector genome titration assays can be used to determine the concentration of AAV vector genomes in a sample. These assays can be quantitative PCR-based or use other techniques like droplet digital PCR (ddPCR).

In one embodiment, preserving the ability of an AAV to transduce or infect a cell in the presence of an anti-AAV neutralising antibody is manifest by an increase in the expression of a transgene inside a cell transduced with AAV in the presence of an anti-AAV neutralising antibody, when the AAV is contacted with albumin, as compared to the expression of a transgene inside a cell transduced with AAV in the presence of an anti-AAV neutralising antibody, when the AAV is not contacted with albumin.

By "transgene", we include the meaning of an exogenous nucleic acid which is incorporated into the genome of the AAV. For example, a transgene could be a reporter, a marker, and/or a therapeutic transgene. However, it is appreciated that the transgene need not include a reporter or marker.

The transgene may comprise any gene of interest, e.g. a nucleic acid sequence that encodes a protein that is desirable for integration into the genome of the AAV. Expression of a transgene may be transient or stable/integrated. Conveniently, the gene of interest may be operably linked to one or more other sequences that are useful for obtaining the desired expression of the gene of interest, such as transcriptional regulatory sequences (e.g. promoters, enhancers, terminators, post transcriptional regulatory elements (PREs)). An example of a PRE is woodchuck hepatitis virus PRE (WRPE). Preferably, the transgene is under the control of a promoter. Suitable promoters include housekeeping or ubiquitous promoters such as CMV, PGK, EF-1$\alpha$1, MND, MCU3, SFFV, CAG and CBh) or tissue specific promoters (such as CD11b, ALB, TBG, MHC, MLC2v, SYN and eTnT promoters). In the context of a therapeutic transgene, tissue specific promoters may be used to ensure that the level of gene expression is in line with the expression of the "damaged" gene or the absent gene that the transgene is intended to replace/augment.

In one embodiment, the transgene encodes a protein reporter. The protein reporter may be a fluorescent protein (such as GFP, dsRed, or mCherry), a luminescent protein (such as luciferase or any other bioluminescent or chemiluminescent molecule), an enzymatic reporter (such as LacZ ($\beta$-galactosidase)), an antibiotic resistance marker (such as a puromycin resistance marker) or a combination thereof.

It will be appreciated that the transgene may encode more than one gene of interest. For example, the transgene may encode a therapeutic protein and a reporter and/or marker protein. The presence of a reporter or marker may be useful to monitor or detect expression of a therapeutic protein in a subject. However, it is also recognised that the expression of therapeutic proteins can be detected without the use of reporter or marker molecules, and so it is not necessary for the transgene to also comprise a reporter or marker.

In one embodiment, preserving the ability of an AAV to transduce or infect a cell in the presence of an anti-AAV neutralising antibody is manifest by an increase in the expression of one or more RNA species inside a cell transduced with AAV in the presence of an anti-AAV neutralising antibody, when the AAV is contacted with albumin, as compared to the expression of one or more RNA species inside a cell transduced with AAV in the presence of an anti-AAV neutralising antibody, when the AAV is not contacted with albumin. Examples of RNA species may include mRNAs, short interfering RNAs, small hairpin RNAs, short guide RNA, long noncoding RNAs or microRNAs.

By "expression" of a transgene or RNA species, we include the meaning of one or more of the following events: (1) production of an RNA template from the viral DNA sequence (e.g., by transcription); (2) processing of an RNA transcript (e.g., by splicing, editing, 5' cap formation, and/or 3' end processing); (3) translation of an RNA into a polypeptide or protein; (4) folding of a polypeptide or protein; and (5) post-translational modification of a polypeptide or protein.

Those skilled in the art would be capable of measuring the expression of a transgene. For example, RNA templates or transcripts can be detected e.g. using polymerase chain reaction and quantified e.g. using Real time (RT) quantitative PCR (qPCR), quantitative reverse transcription PCR, digital PCR or digital drop PCR; polypeptides or proteins can be detected *e.g.* using immunohistochemistry and quantified *e.g.* using flow cytometry, Western blots or enzyme-linked immunosorbent assay (ELISA).

In another embodiment, preserving the ability of an AAV to transduce or infect a cell in the presence of an anti-AAV neutralising antibody is manifest by an increase in the activity of a protein encoded by a transgene inside a cell transduced with AAV in the presence of an anti-AAV neutralising antibody, when the AAV is contacted with albumin, as compared to the activity of a protein encoded by a transgene inside a cell transduced with AAV in the presence of an anti-AAV neutralising antibody, when the AAV is not contacted with albumin.

It will be appreciated that different proteins have different functions, for example: enzymes (*e.g.*, amylase, lipase, pepsin), transport (*e.g.*, hemoglobin), structural support (*e.g.*, actin, tubulin, keratin), chemical signalling (*e.g.*, insulin), and immune response (*e.g.*, antibodies). By "activity of a protein", we include the meaning that the protein is performing its known function. By "increase" in activity, we include the meaning that the protein is performing its known function more optimally or at a higher level. For example, enzymes may process more substrate into product, which increase in product may be detected, and so on.

The level of transduction of a virus can be expressed as a transduction efficiency, namely the proportion of cells that a virus successfully transduces once the virus comes into contact with a population of cells. Thus, by preserving the ability of an AAV to transduce a cell in the presence of an anti-AAV neutralising antibody we include the meaning that the transduction efficiency of the AAV is increased when contacted with albumin, for example relating to the transduction efficiency of the AAV when the AAV has not been contacted with albumin.

The cell may be any cell that is known to be transduced or infected or capable of being transduced or infected with the AAV.

Different subtypes of AAV may have different abilities to transduce different types of cells. For example, AAV2 efficiently transduce both dividing and non-dividing cells, while other subtypes such as AAV5 have a preference for dividing cells. The selectivity is influenced by the viral capsid proteins and their interactions with cell surface receptors.

Hence, it will be appreciated that the cell may be a dividing cell or a non-dividing cell. By a "dividing cell", we include the meaning of a cell that is or (in its current state) is capable of undergoing cell division, such as mitosis or meiosis. Such dividing cells can be totipotent or pluripotent and include stem cells, such as embryonic stem cells, adult stem cells or stem cell in plant meristems. Some cells (*e.g.*, CD34+ stem cells) are 'slowly' dividing cells. By a "non-dividing cell", we include the meaning of a cell that is not undergoing cell division or (in its current state) is incapable of undergoing cell division, such as terminally differentiated cells. Such non-dividing cell can include myocytes, such as cardiomyocytes; primary neurons; glial cells; lymphocytes; macrophages; dendritic cells; epithelial cells; adipocytes.

Under certain conditions, a dividing cell may become a non-dividing cell by entering an inactive stage of mitosis ($G_0$ phase also known as the quiescent stage). Under certain conditions, a non-dividing cell may become a dividing cell.

In one embodiment, the cell is a eukaryotic cell. Preferably the cell is an animal cell. In one embodiment, the animal cell is a mammalian cell, an avian cell or a reptile cell.

In a further embodiment, the cell is any one of an epithelial cell (*e.g.* a nasal epithelial cell), a liver cell, a keratinocyte, a hepatocyte, a muscle cell, a heart cell, an immune cell, a pancreatic cell, an endothelial cell, a stem cell (a haematopoietic stem cell), a bone marrow cell (*e.g.* a bone marrow mesenchymal cell or a bone marrow stromal sell), an osteoblast, a central nervous system cell (*e.g.* a neuron or neuronal cell such as a dopaminergic neuron or a motor neuron, a Purkinje cell, an astrocyte, an ependymal cell or an oligodendrocyte), a cancer cell such as a hepato-cellular carcinoma cell, a lung cell, or a retinal cell.

As mentioned above, different AAV serotypes have distinct tissue tropisms, and so it will be appreciated that the cell in question may be one is known to be targeted by a particular AAV subtype, either preferentially or selectively, or because the AAV subtype has a wide tropism. In other words, when the method or use is directed at preserving the ability of an AAV to transduce or infect a cell in the presence of an anti-AAV neutralising antibody, the cell may be one that is known to be targeted by the AAV. For example, the preferential cellular tropism of AAV1 includes cells of the muscle, heart, CNS, retina and lung tissues; AAV has a wide tropism; the preferential cellular tropism of AAV3 includes hepato-cellular carcinoma cells; the preferential cellular tropism of AAV4 includes cells of the retina, CNS and lung tissues; the preferential cellular tropism of AAV5 includes cells of the lung, liver and CNS tissues; the preferential cellular tropism of AAV6 includes cells of the muscle, heart, lung and bone marrow tissues; the preferential cellular tropism of AAV7 includes cells of the muscle, liver and retina tissues; the preferential cellular tropism of AAV8 includes cells of the muscle, liver, retina, CNS and pancreas tissues; the preferential cellular tropism of AAV9 includes cells of the muscle, heart and CNS tissues; the preferential cellular tropism of AAV10 includes cells of the liver and CNS tissues; and the preferential cellular tropism of AAV12 includes nasal epithelia cells.

In one embodiment, the cell is a cell line. By "cell line" we include the meaning of a defined population of cells that can be maintained in culture for an extended period of time, retaining stability of certain phenotypes and functions. Preferably, the cell line is any of a HeLa cell, a Huh7 cell, a HMEC cell, a human embryonic kidney cell (e.g., HEK293 cell), a HBEC cell (human bronchial epithelial cell), a HepG2 cell, a HT-29 cell, a Jurkat cell, a Saos-2 cell, a U2OS cell, a HUVEC cell or a fibroblast cell.

In one embodiment, the cells are adherent cells. In another embodiment, the cells are ones in suspension in a culture medium.

By contacting the AAV with albumin, we include the meaning that the AAV is contacted with albumin before exposure of the AAV to anti-AAV neutralising antibodies. Without wishing to be bound by any theory, it is thought that the albumin forms a corona around the AAV to protect it from the anti-AAV neutralising antibodies, and so it is preferred that the AAV is contacted with the albumin prior to exposure to anti-AAV neutralising antibodies.

Typically, the AAV is contacted with the albumin for a set period of time, for example prior to exposure to an anti-AAV neutralising antibody. For example, contacting the AAV with albumin may involve incubating the AAV with albumin for a set period of time, for example prior to exposure to an anti-AAV neutralising antibody (e.g., at least 30 minutes, at least 1 hour, 2 hours, 3 hours, 4 hours, 5 hours, 6 hours, 7 hours or 8 hours, at least 24 hours, or at least 36 hours, or at least 2 days). Preferably, the set period of time is from about 30 minutes or 1, 2, 3, 4, 5, 6 or 7 hours to about 1, 2, 3, 4, 5, 6, 7, 8, 16 or 24 hours, such as from about 1 to about 24 hours. More preferably, the set period of time is from about 1 hour to about 10 hours, such as from about 1 hour to about 8 hours.

Generally, when the AAV is contacted with the albumin for a set period of time, for example prior to exposure with an anti-AAV neutralising antibody, the AAV is incubated with albumin at a set temperature, such as at a temperature of from about 2°C to room temperature, such as from about 2, 3, 4, 5, 6, 7, 8, 9, 10, 11, 12, 13, 14, 15, 16, 17, 18, 19 or 20 to about 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33 34 or 35°C, or room temperature. Thus, the AAV may be incubated with albumin at a temperature of from about 4°C to about 10°C. It is preferred is the AAV is incubated with albumin at a temperature of from about 4°C to room temperature, such as about 4°C.

In an embodiment, the AAV is incubated with the albumin for a set period of time (e.g., at least 30 minutes, at least 1 hour, 2 hours, 3 hours, 4 hours, 5 hours, 6 hours, 7 hours or 8 hours, at least 24 hours, or at least 36 hours, or at least 2 days) at a set temperature (e.g., from about 2°C to room temperature, such as from about 2, 3, 4, 5, 6, 7, 8, 9, 10, 11, 12, 13, 14, 15, 16, 17, 18, 19 or 20 to about 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33 34 or 35°C, or room temperature) prior to exposure to an anti-AAV neutralising antibody. Preferably, the AAV is incubated with the albumin for a set period of time from about 1 hour to about 10 hours, more preferably from about 1 hour to about 8 hours such as about 2 hours, at a temperature of from about 4°C to room temperature or about 4°C to 10°C, more preferably at about 4°C. Most preferably, the AAV is incubated with the albumin for about 2 hours at about 4°C prior to exposure to an anti-AAV neutralising antibody. It will be appreciated that the incubation time may vary with the temperature of the incubation. For example, incubation time may increase as the temperature decreases. Also, it will be appreciated that it may be desirable to incubate the AAV with the albumin for a set period of time at a set temperature (e.g. the periods of time and/or temperatures above, such as about 2 hours at about 4°C) and then freeze the mixture containing the AAV and albumin (at about at from about -15°C to about -90°C, e.g., at from about -15°C to about -25°C, e.g., at from about -70°C to about -90°C, e.g., at about -80°C or about -20°C) before subsequent thawing prior to use in transduction or infection of a cell.

In an embodiment, the AAV is any one of AAV serotype 1 (AAV1), AAV2, AAV3, AAV4, AAV5, AAV6, AAV7, AAV8, AAV9, AAV10, AAV11, AAV12, AAV-DJ, AAV-DJ/8, AAV-Rh10, AAV-retro, AAV-PHP.B, AAV8-PHP.eB, and AAV-PHP.S. It is preferred if the AAV is AAV2 or AAV6 or AAV5.

It will be appreciated that the AAV may be one where the genome of the AAV is modified to include a transgene, or an unmodified AAV.

In one embodiment, the AAV does not contain an albumin-binding domain or has not been modified to contain an albumin-binding domain (ABD). By "albumin-binding domain" we include the meaning of a protein domain that is known to bind to albumin, e.g., serum albumin. An example of such a domain is the Streptococcal Protein G albumin-binding domain, such as ABD3. Thus, in a particular embodiment, the AAV has not been modified to include a Streptococcal Protein G albumin-binding domain, such as ABD3.

By an anti-AAV neutralising antibody we include the meaning of any antibody that specifically binds to AAV and inhibits an activity of the AAV. Preferably, the anti-AAV neutralising antibody is one that specifically binds to AAV and reduces or inhibits the ability of the AAV to infect or transduce a cell, for example by blocking the interaction between the AAV and a cell surface receptor. The anti-AAV neutralising antibody may be one that specifically binds to one or more of AAV serotype 1 (AAV1), AAV2, AAV3, AAV4, AAV5, AAV6, AAV7, AAV8, AAV9, AAV10, AAV11, AAV12, AAV-DJ, AAV-DJ/8, AAV-Rh10, AAV-retro, AAV-PHP.B, AAV8-PHP.eB, and AAV-PHP.S.

In a preferred embodiment, the anti-AAV neutralising antibody is one that specifically binds to AAV5, or is one that specifically binds to AAV3, AAV2 and AAV6, or is one that specifically binds to AAV2. Examples of anti-AAV neutralising antibody include anti-AAV-2 mouse monoclonal antibody from Origene (BM5010B) and anti-AAV-2 from Progen (1055). Anti-AAV1, anti-AAV5, anti-AAV6, anti-AAV7, anti-AAV8 and anti-AAV9 nAbs have been found to be prevalent in human serum samples (Calecedo and Wilson, 2013 and Bashirians *et al.,* 2022) and so it will be appreciated that the neutralising antibody may also be any of an AAV1, anti-AAV5, anti-AAV6, anti-AAV7, anti-AAV8 and anti-AAV9 antibody.

The term 'albumin' as described herein includes the meaning of a protein having the same and/or very similar tertiary structure as human serum albumin (HSA; in particular SEQ ID NO: 2) or HSA domains and has similar properties of HSA or the relevant domains. Similar tertiary structures are, for example, the structures of the albumins from species other than human, e.g. non-human primate albumin, (such as chimpanzee albumin (e.g. predicted sequence GenBank XP_517233.2), gorilla albumin or macaque albumin (e.g. GenBank NP_001182578)), rodent albumin (such as hamster albumin (e.g. GenBank A6YF56), guinea pig albumin (e.g. UniProt Q6WDN9-1), mouse albumin (e.g. GenBank AAH49971 or UniProt P07724-1 Version 3) and rat albumin (e.g. GenBank AAH85359 or UniProt P02770-1 Version 2)), bovine albumin (such as cow albumin (e.g. UniProt P02769-1)), equine albumin (such as horse albumin (e.g. UniProt P35747-1) or donkey albumin (e.g. UniProt Q5XLE4-1)), rabbit albumin (e.g. UniProt P49065-1 Version 2), goat albumin (e.g. GenBank ACF10391), sheep albumin (e.g. UniProt P14639-1), dog albumin (e.g. NCBI NP_001003026), chicken albumin (e.g. UniProt P19121-1 Version 2) and pig albumin (e.g. UniProt P08835-1 Version 2), or any one of SEQ ID NOs: 4 to 19 of WO 2013/006675, which are incorporated herein by reference. All of these albumins are included in the scope of the present invention. Mature forms of albumin (e.g. forms where all post-translational modifications and/or processing steps have been completed) are particularly preferred, and the skilled person is able to identify mature forms using publicly available information such as protein databanks and/or by using signal peptide recognition software such as SignalP (e.g. SignalP (Nielsen et al, 1997, Protein Engineering 10(1): 1-6)). SignalP Version 4.0 is preferred (Petersen et al, 2011, Nat Methods 8(10):785-786). An albumin preparation for use in the methods and uses of the present invention may comprise one or more (several) albumins.

Some of the major properties of albumin are i) its ability to regulate plasma volume, ii) a long plasma half-life of around 19 days $\pm$ 5 days, iii) ligand binding, e.g. binding of endogenous molecules such as acidic, lipophilic compounds including bilirubin fatty acids, hemin and thyroxine (see also Table 1 of Kragh-Hansen et al, 2002, Biol Pharm Bull 25(6):695-704, hereby incorporated by reference), iv) binding of small organic compounds with acidic or electronegative features, e.g. drugs such as warfarin, diazepam, ibuprofen and paclitaxel (see also 1 of Kragh-Hansen et al, 2002, Biol Pharm Bull 25(6):695-704, hereby incorporated by reference). Not all of these properties need to be fulfilled in order to characterise a protein or fragment as an albumin. If a fragment, for example, does not comprise a domain responsible for binding certain ligands or organic compounds, the variant of such a fragment is not expected to have these properties either.

The term 'albumin' includes fragments, variants, and/or derivatives such as fusions and/or conjugations of an albumin or of an albumin variant. The term "fusion" includes the meaning a genetic fusion of albumin (or a variant or fragment thereof) and a non-albumin protein or peptide. The non-albumin protein or peptide may be a therapeutic, prophylactic, or diagnostic protein or peptide. Examples of albumin fusions are provided in EP624195, WO 2001/079271, WO 2003/059934, WO 2003/060071, WO 2011051489, WO 2011/124718 and EP11164955 (each incorporated herein by reference in their entirety).

By 'variant' we include the meaning of a polypeptide derived from a parent albumin comprising an alteration, i.e. a substitution, insertion and/or deletion, at one or more (several) positions. Such alterations may be referred to as "mutations". It is preferred if the parent albumin is a mature albumin, for example one without the leader sequence. A substitution includes the meaning of a replacement of an amino acid occupying a position with a different amino acid; a deletion includes the meaning of the removal of an amino acid occupying a position; and an insertion includes the meaning of adding amino acids (e.g. 1-3 amino acids) adjacent to an amino acid occupying a position. For example, the altered polypeptide (variant) can be obtained through human intervention by modification of the polynucleotide sequence encoding the parent albumin. The mature form of the albumin sequence may be encoded by the nucleotide sequence SEQ ID NO: 1. The variant albumin is preferably at least 70%, preferably at least 75%, more preferably at least 80%, more preferably at least 85%, even more preferably at least 90%, most preferably at least 91%, at least 92%, at least 93%, at least 94%, at least 95%, at least 96%, at least 97%, at least 98%, at least 99%, or 100% identical to SEQ ID NO: 2.

Preferably, the albumin has at least 70% sequence identity to a mammalian albumin, preferably from an albumin selected from the group consisting of human (SEQ ID NO: 2), mouse (GenBank AAH49971 or UniProt P07724-1 Version 3), rat (GenBank AAH85359 or UniProt P02770-1 Version 2), macaque (GenBank NP_001182578), bovine (such as cow albumin (e.g. UniProt P02769-1), pig (UniProt P08835-1 Version 2), horse (UniProt P35747-1), rabbit (UniProt P49065-1 Version 2), dog (Canis lupus familiaris, Accession number NP_001003026, where residues 1 to 18 are the signal peptide, 19 to 24 are the propeptide and residues 25 to 608 are the mature sequence) or guinea pig (Cavia porcellus, UniProt Q6WDN9-1 where residues 1 to 18 are the signal peptide and 25 to 608 are the mature sequence) albumin. Preferably, the albumin has at least 70% sequence identity to a mammalian albumin selected from any of HSA (mature sequence provided in SEQ ID NO: 2, immature sequence provided in SEQ ID NO: 10 in which residues 1 to 18 of the immature sequence are the signal peptide, 19 to 24 are the propeptide and residues 25 to 609 are the mature sequence), mouse serum albumin (SEQ ID NO: 3 where residues 1 to 18 are the signal peptide, 19 to 24 are the propeptide and residues 25 to 608 are the mature sequence), rat serum albumin (SEQ ID NO: 4 where residues 1 to 18 are the signal peptide, 19 to 24 are the propeptide and residues 25 to 608 are the mature sequence), macaque serum albumin (SEQ ID NO: 5 where residues 1 to 18 are the signal peptide, 19 to 24 are the propeptide and residues 25 to 608 are the mature sequence), bovine serum albumin (SEQ ID NO: 6 where residues 1 to 18 are the signal peptide, 19 to 24 are the propeptide and residues 25 to 607 are the mature sequence), pig serum albumin (SEQ ID NO: 7 where residues 1 to 18 are the signal peptide, 19 to 24 are the propeptide and residues 25 to 607 are the mature sequence), horse serum albumin (SEQ ID NO: 8 where residues 1 to 18 are the signal peptide, 19 to 24 are the propeptide and residues 25 to 607 are the mature sequence), or rabbit serum albumin (SEQ ID NO: 9 where residues 1 to 18 are the signal peptide, 19 to 24 are the propeptide and residues 25 to 608 are the mature sequence). It is preferred if the albumin variant is a variant of a mature form of albumin. For example, when the parent albumin is mouse serum albumin, preferably, the variant has at least 70% sequence identity to residues 25 to 608 of SEQ ID NO: 3, and so on for the other mammalian sequences disclosed herein. Preferably, the albumin has at least 70% sequence identity to HSA (SEQ ID NO: 2), more preferably from 70, 75, 80, 85, 90, 91, 92, 93, 94, 95, 96, 97, 98, 99, 99.2, 99.4, 99.6, or 99.8 to 75, 80, 85, 90, 91, 92, 93, 94, 95, 96, 97, 98, 99, 99.2, 99.4, 99.6, 99.8 or 100% identity to HSA (SEQ ID NO: 2). For example, a preferred albumin has, at most, 12, 11, 10, 9, 8, 7, 6, 5, 4, 3, 2 or 1 amino acid mutations relative to wild-type HSA (SEQ ID NO: 2).

Preferably, the albumin comprises at least 175 contiguous amino acids from an albumin having at least 70% sequence identity to HSA (SEQ ID NO: 2), such as from 175, 200, 225, 250, 275, 300, 325, 350, 375, 400, 425, 450, 475, 500, 525, 550 or 575 to 200, 225, 250, 275, 300, 325, 350, 375, 400, 425, 450, 475, 500, 525, 550, 575 or 580 amino acids. A fragment may comprise, consist of or substantially correspond to one or more (e.g. several) domains of albumin such as HSA (SEQ ID NO: 2), or variant thereof, such as amino acids corresponding to Domain I (residues 1 to 194 ± 1, 2, 3, 4, 5, 6, 7, 8, 9, 10, 11, 12, 13, 14 or 15 amino acids), Domain II (residues 192 to 387 ± 1, 2, 3, 4, 5, 6, 7, 8, 9, 10, 11, 12, 13, 14 or 15 amino acids) or Domain III (residues 381 to 585 ± 1, 2, 3, 4, 5, 6, 7, 8, 9, 10, 11, 12, 13, 14 or 15 amino acids).

Typically, the variant albumin maintains at least one of the major properties of the parent albumin or a similar tertiary structure as HSA. For the purposes of the present invention, the sequence identity between two amino acid sequences may be determined using the Needleman-Wunsch algorithm (Needleman & Wunsch, 1970, J Mol Biol 48(3):443-453) as implemented in the Needle program of the EMBOSS package (EMBOSS: The European Molecular Biology Open Software Suite, Rice et al, 2000, Trends Genet 16(6):276-277), preferably version 5.0.0 or later. The typical parameters used are gap open penalty of 10, gap extension penalty of 0.5, and the EBLOSUM62 (EMBOSS version of BLOSUM62) substitution matrix. The output of Needle labelled "longest identity" (obtained using the -nobrief option) may be used as the percent identity and may be calculated as follows: (Identical Residues × 100)/(Length of Alignment- Total Number of Gaps in Alignment).

Examples of albumin variants include those described in WO 2011/051489, WO 2011/124718, WO 2012/059486, WO 2012/150319, WO 2014/072481, WO 2013/135896, WO 2015/036579, WO 2010/092135, WO 2013/075066, WO 2014/179657, WO 2009/126920, WO 2010/059315, WO 2011/103076, WO 2012/112188, WO 2015/063611, and WO 2017/029407 (the contents of which are incorporated herein by reference in their entirety, and in particular references to albumin variants).

The term 'parent' or 'parent albumin' as described herein includes the meaning of a human or other animal (e.g. mammalian) albumin. Preferably, the other mammalian albumin is an albumin from a clinically relevant animal such as a mouse, rat, rabbit, dog or guinea pig. The parent may be a naturally occurring (wild-type) polypeptide or an allele thereof or a variant as described above.

The term 'wild-type' (WT) albumin as described herein includes the meaning of an albumin having the same amino acid sequence as the predominant allelic variant of albumins naturally found in an animal or in a human being. SEQ ID NO: 2 is an example of a wild-type albumin, being wild-type albumin from Homo sapiens.

In one embodiment, the albumin is ovalbumin. Ovalbumin (OVA) or albumen refers to the main protein found in egg white. In one embodiment, the egg is an avian egg, such as a chicken egg. In one embodiment, the ovalbumin is chicken ovalbumin (UniProtKB/Swiss-Prot: P01012.2), according to SEQ ID NO: 11.

In one embodiment, the ovalbumin is a variant of a wild-type ovalbumin, optionally wherein the variant ovalbumin has at least 70%, 80%, 85%, 90%, 91%, 92%, 93%, 94%, 95%, 96%, 97%, 98% or 99% sequence identity with the wild-type ovalbumin, such as at least 70%, 80%, 85%, 90%, 91%, 92%, 93%, 94%, 95%, 96%, 97%, 98% or 99% sequence identity with wild-type chicken ovalbumin according to SEQ ID NO: 11.

In one embodiment, the ovalbumin has at least 70% sequence identity to wild-type chicken ovalbumin (SEQ ID NO: 11), more preferably from 70, 75, 80, 85, 90, 91, 92, 93, 94, 95, 96, 97, 98, 99, 99.2, 99.4, 99.6, or 99.8 to 75, 80, 85, 90, 91, 92, 93, 94, 95, 96, 97, 98, 99, 99.2, 99.4, 99.6, 99.8 or 100% identity to wild-type chicken ovalbumin (SEQ ID NO: 11). For example, the ovalbumin may have 12, 11, 10, 9, 8, 7, 6, 5, 4, 3, 2 or 1 amino acid mutations relative to wild-type chicken ovalbumin (SEQ ID NO: 11).

Preferably, the ovalbumin has 100% identity to wild-type chicken ovalbumin (SEQ ID NO: 11).

In one embodiment, the albumin is recombinant albumin. In another embodiment, the albumin is a serum albumin. In one embodiment, the albumin is recombinant human albumin or human serum albumin. The recombinant human albumin and the human serum albumin may have similar abilities at enhancing transduction. The recombinant ovalbumin and the egg-derived ovalbumin may have similar abilities at enhancing transduction.

In one embodiment, the albumin is sourced from a recombinant source or is sourced from serum. By "sourced from a recombinant source", we include the meaning that the albumin may be sourced from a recombinant organism such as a recombinant microorganism, recombinant plant or recombinant animal.

Since some users prefer animal-free ingredients, it is more preferred that the albumin is sourced from a non-animal recombinant source, such as a recombinant microorganism or recombinant plant. Preferred organisms include prokaryotes and, more preferably, eukaryotes such as animals, plants, fungi or yeasts, for example, but not limited to, the following species in which albumins have been successfully expressed as recombinant proteins, for example see the following citations which are incorporated herein by reference:

- fungi (including but not limited to *Aspergillus* (WO06066595), *Kluyveromyces* (Fleer 1991, Bio/technology 9, 968-975), *Pichia* (Kobayashi 1998 Therapeutic Apheresis 2, 257-262) and *Saccharomyces* (Sleep 1990, Bio/technology 8, 42-46))
- animals (Barash 1993, Transgenic Research 2, 266-276)
- plants (including but not limited to potato and tobacco (Sijmons 1990, Biotechnology 8, 217 and Farran 2002, Transgenic Research 11, 337-346) and rice *e.g., Oryza sativa*)
- mammalian cells such as CHO and HEK
- prokaryotes (Pandjaitab 2000, J. Allergy Clin. Immunol. 105, 279-285)) *e.g., E. coli* (EP73646).

In one embodiment, the albumin is: a) yeast-derived albumin, optionally wherein the yeast is *Pichia* such as *Pichia pastoris, Saccharomyces* such as *Saccharomyces cerevisiae, Candida* such as *Candida utilis, Kluyveromyces* such as *Kluyveromyces lactis* or *Kluyveromyces marxianus, Hansenula polymorpha, Schizosaccharomyces pombe, Yarrowia lipolytica, Arxula adeninivorans,* or Zygosaccharomyces such as *Zygosaccharomyces bailii*; or b) a plant-derived albumin, optionally a rice-derived albumin. Preferably, the albumin is derived from *Saccharomyces,* most preferably Saccharomyces *cerevisiae* (Celik and Calik (Biotechnology Advances, 2012, 30(5): 1108-1118), Gunduz Ergun et al, "Established and Upcoming Yeast Expression Systems" in "Recombinant Protein Production in Yeast", 2019, volume 1923, pages 1-74. Editors: Gasser and Mattanovich, Publisher: Humana New York, NY.). Particularly preferred examples of albumins (*e.g.*, recombinant human albumin) include those manufactured in yeast, particularly in Saccharomyces cerevisiae, such as the following known commercial presentations of recombinant yeast-derived albumin: Recombumin® Prime (formerly Recombumin®), Recombumin® Elite (formerly AlbIX®), Recombumin® Alpha (formerly Albucult®) (all sourced from Sartorius Albumedix Limited or predecessor companies such as Albumedix Ltd or Novozymes Biopharma DK A/S), or any preparation that is similar thereto.

In a preferred embodiment, the albumin is provided in a form that is substantially free or free of other components such as any one or more of an antibody, a peptide, a protein, a lipid, a carbohydrate, a small molecule or an active pharmaceutical ingredient (API) or biopharmaceutical (e.g., a biopharmaceutical that has been classified as Class I, II, III or IV according to the Biopharmaceutics Classification Scheme (BCS) that was first proposed by Amidon et al (Pharm Res. 1995, 12(3):413-420)). In other words, when the albumin is contacted with the AAV the albumin is provided free of such other components and is optionally provided with one or more of a buffer and an excipient.

In an embodiment of the first aspect of the invention, the AAV is contacted with the albumin at an albumin concentration of from about 1 mg/mL to about 400 mg/mL, such as from about 2.5, 5, 7.5, 10, 15, 20, 25, 30, 35, 40, 45, 50, 55, 60, 65, 70, 75, 80, 85, 90, 95, 100, 105, 110, 115, 120, 125, 130, 135, 140, 145, 150, 160, 170, 180, 190, 200, 210, 220, 230, 235, 240, 245 or 250 mg/mL to about 10, 15, 20, 25, 30, 35, 40, 45, 50, 55, 60, 65, 70, 75, 80, 85, 90, 95, 100, 110, 115, 120, 125, 130, 135, 140, 145, 150, 160, 170, 180, 190, 200, 210, 220, 230, 240, 250, 260, 270, 280, 290, 300, 310, 320, 330, 340, 350, 355, 360, 365, 370, 375, 380, 385, 390, 392.5, 395, 397.5, 398, 399 or 400 mg/mL. Preferably, the AAV is contacted with the albumin at an albumin concentration of from about 1 to about 300 mg/mL, such as from about 25 mg/mL to about 300 mg/mL, or from about 50 mg/mL to about 300 mg/mL, or from about 75 mg/mL to about 300 mg/mL, or from about 100 mg/mL to about 300 mg/mL, or from about 100 mg/mL to about 200 mg/mL, or from about 200 mg/mL to about 300 mg/mL or from about 150 to about 250 mg/mL or from about 150 to about 200 mg/mL, or from about 200 mg/mL to about 250 mg/mL. For example, the AAV may be contacted with the albumin at an albumin concentration of from about 25, 50, 75, 100, 110, 120, 130, 140, 150, 160, 170, 180, 190, 200, 210, 220, 230, 240 or 250 mg/mL to about 50, 75, 100, 110, 120, 130, 140, 150, 160, 170, 180, 190, 200, 210, 220, 230, 240, 250, 260, 270, 280, 290 or 300 mg/mL. It is preferred if the AAV is contacted with the albumin at an albumin concentration of from about 25 mg/mL to about 250 mg/mL. More preferably, the AAV is contacted with the albumin at an albumin concentration of from about 175 to about 225 mg/mL such as about 200 mg/mL.

It will be appreciated that the method or use may be carried out *ex vivo* or *in vitro or in vivo.*

Typically, following contacting the AAV with albumin when the method or use is being carried out *in vitro* or *ex vivo,* the albumin and AAV mixture is added to cells that are located in a receptacle or vessel such as a well of a plate such as a multi-well plate. It will be appreciated that the cells may be in contact with AAV neutralising antibodies before the albumin and AAV mixture is added to them. For example, if the cell culture medium contains serum from animals or humans, it may carry pre-existing AAV neutralising antibodies, or the use of specific reagents or supplements may introduce AAV neutralising antibodies. It will also be appreciated that once the albumin/AAV mixture is added to the cells, the albumin may be diluted, and so the albumin concentration in a composition comprising the cells, AAV and albumin (e.g., the composition in the well of a multi-well plate) may be lower. For example, as described in the Examples, when 16 μL of 200 mg/mL albumin is added to the required amount of AAV to form a pre-mix, which pre-mix is then added to 100 μL of media in contact with the cells, the albumin concentration in a composition comprising the cells, AAV and albumin is reduced to 27.6 mg/mL. Thus, in one embodiment, the composition comprising the albumin, AAV and cells (e.g., the composition in the well of a multi-well plate) contains albumin at a concentration of from about 0.5 mg/mL to about 50 mg/mL, such as from about 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34 or 35 to about 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50 mg/mL. Preferably, the albumin concentration in a composition comprising the cells, AAV and albumin (e.g., the composition in the well of a multi-well plate) is at a concentration of from about 2 to about 40 mg/mL, more preferably about 5 to about 35 mg/mL, such as about 10 to about 30 mg/mL, most preferably about 25 to about 30 mg/mL, such as about 27 mg/mL.

In one embodiment, the AAV is present at a multiplicity of infection (MOI) range of from about 0.1 to about $10^8$, such as from about 1, 10, $10^2$, $10^3$, $10^4$, $10^5$, $10^6$ or $10^7$ to about $10^3$, $10^4$, $10^5$, $10^6$ or $10^7$ or $10^8$. Preferably, the AAV is present at an MOI of from about $10^2$ to about $10^7$, and most preferably the AAV is present at an MOI of from about $10^3$ to about $10^6$.

By "multiplicity of infection" or "MOI", we include the meaning of the ratio of number of virus particles to number of target cells present. In general, as the MOI increases, the percentage of cells infected with at least one viral particle also increases. Those skilled in the art would be capable of determining MOI, as number of infectious virus particles

$$\frac{\text{number of infectious virus particles}}{\text{number of target cells}}$$

. The optimum MOI can be determined experimentally by those skilled in the art.

In one embodiment, when contacting the AAV with albumin, one or both of the AAV and the albumin may be provided in a buffer and mixed together. Alternatively, the AAV and albumin may both be added to a buffer.

In one embodiment, the buffer has a buffering capacity from about pH 6.0 to about pH 8.0, such as from about pH 6.0, 6.1, 6.2, 6.3, 6.4, 6.5, 6.6, 6.7, 6.8, 6.9, 7.0, 7.1 to about pH 6.7, 6.8, 6.9, 7.0, 7.1, 7.2, 7.3, 7.4, 7.5, 7.6, 7.7, 7.8, 7.9, 8.0.

In one embodiment, the buffer is any of phosphate-buffered saline (PBS), Hanks buffered salt solution, Earle's balanced salt solution, Tyrode's solution, MOPS buffer, HEPES buffer or any isotonic solutions used for injection, such as Plasma-Lyte®, e.g., Plasma-Lyte® 148, or Ringer's lactate. Preferably, the buffer is PBS.

When a buffer is present, the buffer concentration may be from about 1, 5, 10, 15, 20, 25, 30, 35, 40, 45, 50, 55, 60, 65, 70, 75, 80, 85, 90, 95, or 100 mM to about 5, 10, 15, 20, 25, 30, 35, 40, 45, 50, 55, 60, 65, 70, 75, 80, 85, 90, 95, 100, 150, 200 or 250 mM. A buffer concentration from about 10 mM to about 200 mM is preferred, particularly about 150 mM, particularly wherein the buffer is PBS.

The albumin that contacts the AAV may conveniently be provided in the form of an albumin formulation. The formulation is typically a liquid albumin formulation although it is appreciated that the formulation may be a dried formulation such as a freeze-dried formulation. Such a dried formulation may be added to a solution containing the AAV.

In one embodiment, the albumin which is used to contact the AAV is provided in the form of a formulation, and suitable formulations are any of the group consisting of: (a) from 13 to 16 mM, preferably about 14.5 mM, of sodium, from 2.9 to 3.5 mM, preferably about 3.2 mM, of octanoate, from 1 to 2 mg/L, preferably from 1 to 1.5 mg/L, of Polysorbate 80, from 19 to 21 mg/mL, preferably about 20 mg/mL, of albumin (weight/volume (w/v)), wherein the pH is 6.7 to 7.3, preferably about 7; (b) from 12 to 16 mM, preferably about 14.5 mM, of sodium, from 0.4 to 1.2 mM, preferably about 0.8 mM, of octanoate, from 0 to 5 mg/L, preferably about 2.5 to 4.5 mg/L, of Polysorbate 80, from 9.5 to 10.5 mg/mL, preferably about 10 mg/mL, of albumin (w/v), wherein the pH is 6.4 to 7.4, preferably about 7; (c) from 12 to 16 mM, preferably about 14.5 mM, of sodium, from 0.8 to 2.4 mM, preferably about 1.6 mM, of octanoate, from 0 to 10 mg/L, preferably from 5 to 7 mg/L, of Polysorbate 80, from 19 to 21 mg/mL, preferably about 20 mg/mL, of albumin (w/v), wherein the pH is 6.4 to 7.4, preferably about 7; (d) from 20 to 30 mM, preferably from 23 to 26 mM, of sodium, from 0 to 0.3 mM, preferably from 0 to 0.1 mM of octanoate, from 9.5 to 10.5 mg/mL, preferably about 10 mg/mL, of albumin (w/v), wherein the pH is from 6 to 7, preferably about 6.5.

In another embodiment, suitable formulations are any of the group consisting of: (a) from 130 to 160 mM, preferably about 145 mM, of sodium, from 29 to 35 mM, preferably about 32 mM, of octanoate, from 10 to 20 mg/L, preferably from 10 to 15 mg/L, of Polysorbate 80, from 190 to 210 mg/mL, preferably about 200 mg/mL, of albumin (weight/volume (w/v)), wherein the pH is 6.7 to 7.3, preferably about 7; (b) from 120 to 160 mM, preferably about 145 mM, of sodium, from 4 to 12 mM, preferably about 8 mM, of octanoate, from 0 to 50 mg/L, preferably from 25 to 45 mg/L, of Polysorbate 80, from 95 to 105 mg/mL, preferably about 100 mg/mL, of albumin (w/v), wherein the pH is 6.4 to 7.4, preferably about 7; (c) from 120 to 160 mM, preferably about 145 mM, of sodium, from 8 to 24 mM, preferably about 16 mM, of octanoate, from 0 to 100 mg/L, preferably from 50 to 70 mg/L, of Polysorbate 80, from 190 to 210 mg/mL, preferably about 200 mg/mL, of albumin (w/v), wherein the pH is 6.4 to 7.4, preferably about 7; (d) from 200 to 300 mM, preferably from 230 to 260 mM, of sodium, from 0 to 3 mM, preferably from 0 to 1 mM of octanoate, from 95 to 105 mg/mL, preferably about 100 mg/mL, of albumin (w/v), wherein the pH is from 6 to 7, preferably about 6.5.

In a further embodiment, suitable formulations are any of the group consisting of (a) from 6.5 to 8 mM, preferably about 7.25 mM, of sodium, from 1.45 to 1.75 mM, preferably about 1.6 mM, of octanoate, from 0.5 to 1 mg/L, preferably from 0.5 to 0.75 mg/L, of Polysorbate 80, from 9.5 to 10.5 mg/mL, preferably about 10 mg/mL, of albumin (weight/volume (w/v)), wherein the pH is 6.7 to 7.3, preferably about 7; (b) from 6 to 8 mM, preferably about 7.25 mM, of sodium, from 0.2 to 0.6 mM, preferably about 0.4 mM, of octanoate, from 0 to 2.5 mg/L, preferably from 1.25 to 2.25 mg/L, of Polysorbate 80, from 4.75 to 5.25 mg/mL, preferably about 5 mg/mL, of albumin (w/v), wherein the pH is 6.4 to 7.4, preferably about 7; (c) from 6 to 8 mM, preferably about 7.25 mM, of sodium, from 0.4 to 1.2 mM, preferably about 0.8 mM, of octanoate, from 0 to 5 mg/L, preferably about 2.5 to 3.5 mg/L, of Polysorbate 80, from 9.5 to 10.5 mg/mL, preferably about 10 mg/mL, of albumin (w/v), wherein the pH is 6.4 to 7.4, preferably 7; (d) from 10 to 15 mM, preferably from 11.5 to 13 mM, of sodium, from 0 to 0.15 mM, preferably from 0 to 0.05 mM of octanoate, from 4.75 to 5.25 mg/mL, preferably about 5 mg/mL, of albumin (w/v), wherein the pH is from 6 to 7, preferably about 6.5.

In one embodiment, the albumin formulation contains albumin at a concentration from 4.75 to 400 mg/mL, preferably from 10 to 250 mg/mL mM, such as from 95 to 210 mg/mL, more preferably from 10 to 100 mg/mL, most preferably from about 10 to about 20 mg/mL.

In one embodiment, the albumin formulation contains sodium at a concentration from 1 to 300 mM (e.g., from 120-300 mM), preferably from 10 to 50 mM, more preferably from 12 to 30 mM, most preferably about 25 mM.

In one embodiment, the albumin formulation contains sodium at from 0.25 mM sodium per gram albumin to 200 mM sodium per gram albumin, for example from 0.25, 0.3, 0.35, 0.3625, 0.4, 0.45, 0.5, 0.6, 0.7, 0.725, 0.75, 0.8, 0.9, 1, 1.45, 2, 2.9, 3, 4, 5, 5.8, 6, 7, 7.25, 8, 9, 10, 15, 20, 25, 50, 75, 100, 125, 150 or 175 to 0.3, 0.35, 0.3625, 0.4, 0.45, 0.5, 0.6, 0.7, 0.725, 0.75, 0.8, 0.9, 1, 2, 3, 4, 5, 6, 7, 8, 9, 10, 15, 20, 25, 50, 75, 100, 125, 145, 150, 175 or 200 mM sodium per gram albumin, preferably from 0.5 to 6 mM sodium per gram albumin, more preferably from to 0.5 to 3 mM sodium per gram albumin, such as from 0.725 to 2.9 mM sodium per gram albumin.

In one embodiment, the albumin formulation contains octanoate at a concentration from 0 to 35 mM, preferably from 0 to 5 mM, more preferably from 0 to 3.5 mM, most preferably about 0.1 mM.

In one embodiment, the albumin formulation contains octanoate at from 0.01 to 25 mM octanoate per gram albumin, for example from 0.01, 0.02, 0.03, 0.04, 0.05, 0.06, 0.07, 0.08, 0.09, 0.1, 0.11, 0.12, 0.13, 0.14, 0.15, 0.16, 0.17, 0.18, 0.19, 0.2, 0.21, 0.22, 0.23, 0.24, 0.25, 0.26, 0.27, 0.28, 0.29, 0.3, 0.31, 0.32, 0.33, 0.34, 0.35, 0.36, 0.37, 0.38, 0.39, 0.4, 0.41, 0.42, 0.43, 0.44, 0.45, 0.46, 0.47, 0.48, 0.49, 0.5, 0.6, 0.7, 0.8, 0.9, 1, 1.1, 1.2, 1.3, 1.4, 1.5, 1.6, 1.7, 1.8, 1.9, 2, 3, 4, 5, 6, 7, 8, 9, 10, 11, 12, 13, 14, 15, 16, 17, 18, 19, 20, 21, 22, 23, 24 mM octanoate per gram albumin, to 0.01, 0.02, 0.03, 0.04, 0.05, 0.06, 0.07, 0.08, 0.09, 0.1, 0.11, 0.12, 0.13, 0.14, 0.15, 0.16, 0.17, 0.18, 0.19, 0.2, 0.21, 0.22, 0.23, 0.24, 0.25, 0.26, 0.27, 0.28, 0.29, 0.3, 0.31, 0.32, 0.33, 0.34, 0.35, 0.36, 0.37, 0.38, 0.39, 0.4, 0.41, 0.42, 0.43, 0.44, 0.45, 0.46, 0.47, 0.48, 0.49, 0.5, 0.6, 0.7, 0.8, 0.9, 1, 1.1, 1.2, 1.3, 1.4, 1.5, 1.6, 1.7, 1.8, 1.9, 2, 3, 4, 5, 6, 7, 8, 9, 10, 11, 12, 13, 14, 15, 16, 17, 18, 19, 20, 21, 22, 23, 24, 25 mM octanoate per gram albumin, preferably from 0.05 to 0.7 mM octanoate per gram albumin, more preferably from to 0.05 to 0.4 mM octanoate per gram albumin, such as from 0.08 to 0.32 mM octanoate per gram albumin.

In one embodiment, the albumin formulation contains Polysorbate 80 at a concentration from 0 to 100 mg/mL, preferably from 0 to 10 mg/mL, more preferably from 0 to 5 mg/mL, most preferably about 2.5 mg/mL.

In one embodiment, the albumin formulation contains a detergent, such as a Polysorbate, such as Polysorbate 80, at from 0.1, 0.125, 0.15, 0.175, 0.2, 0.225, 0.25, 0.275, 0.3, 0.4, 0.5, 0.6, 0.7, 0.8, 0.9, 1, 1.5, 2, 2.5, 3, 3.5, 4, 4.5, 5, 6, 7, 8, 9, 10, 15, 20, 25, 30, 35, 40, 45, 50, 55, 60, 65, 70 mg detergent per gram albumin, to 0.1, 0.125, 0.15, 0.175, 0.2, 0.225, 0.25, 0.275, 0.3, 0.4, 0.5, 0.6, 0.7, 0.8, 0.9, 1, 1.5, 2, 2.5, 3, 3.5, 4, 4.5, 5, 6, 7, 8, 9, 10, 15, 20, 25, 30, 35, 40, 45, 50, 55, 60, 65, 70, 75 mg detergent per gram albumin, preferably from 0.2 to 2.5 gram detergent per gram albumin, more preferably from 0.2 to 1.25 mg detergent per gram albumin, such as from 0.25 to 1 mg detergent per gram albumin.

A second aspect of the invention provides a method of improving the efficacy of an AAV-based therapy, the method comprising contacting an AAV with albumin prior to administering the AAV to a subject. Similarly, the invention includes the use of albumin to improving the efficacy of an AAV-based therapy, wherein the use involves contacting the AAV-based therapy with albumin prior to administering the AAV to a subject.

A third aspect of the invention provides a method of reducing the neutralisation of an adeno-associated virus (AAV) based therapy by an anti-AAV neutralising antibody, the method comprising contacting an AAV with albumin prior to administering the AAV to a subject. Similarly, the invention includes the use of albumin to reduce the neutralisation of an AAV-based therapy by an anti-AAV neutralising antibody, wherein the use involves contacting the AAV-based therapy with albumin prior to administering the AAV to a subject.

It will be appreciated that in accordance with the second and third aspects of the invention, the AAV is an AAV which is intended to have a therapeutic effect. Similarly, it will be appreciated that the AAV in the first aspect of the invention may be one that has a therapeutic effect.

By "AAV-based therapy", we include the meaning of a therapy that is based on AAV to treat and/or prevent a disease or condition that would benefit from the therapy. For example, the AAV-based therapy may be a gene-based therapy, wherein the AAV is used as a vector to transfer genetic material (e.g. a particular gene or part thereof) to a cell or tissue so as to prevent and/or treat a disease or condition. An example of an AAV-based gene therapy is Luxturna which targets Leber Congenital Amaurosis (LCA), a genetic condition causing severe vision impairment or blindness from birth. Luxturna uses an AAV vector to deliver a functional RPE65 gene directly to the retina to improve vision. Another example is Zolgensma which is used to treat spinal muscular atrophy. Zolgensma delivers a functional copy of the SMN1 gene. Other AAV-based therapies include Glybera (for Lipoprotein lipase deficiency), Upstaza (for AADC), Hemgenix (for Haemophilia B), Elvidys (for DMD), Beqvez (for Congenital Factor IX deficiency) and Roctavian (for Haemophilia A).

Such therapies can be used to treat and/or prevent diseases and/or conditions. In one embodiment, the disease or condition is a genetic disease or condition. In another embodiment, the disease or condition is a sporadic disease or condition. Examples of such diseases or conditions include but are not limited to as cancer (such as lymphoma, leukemia or multiple myeloma), lysosomal storage diseases, β-thalassemia, cerebral adrenoleukodystrophy, sickle cell disease, haemophilia, Fanconi anemia, metachromatic leukodystrophy and adrenoleukodystrophy, Wiskott-Aldrich syndrome, Severe combined immunodeficiency (SCID), X-linked severe combined immunodeficiency (XL-SCID), adenosine deaminase severe combined immunodeficiency (ADA-SCID), X-linked chronic granulomatous disease (XL-CGD), Parkinson's disease, macular degeneration.

By "gene therapy", we include the meaning of a technique that modifies a subject's genes to treat, cure or prevent a disease or disorder. Gene therapies can work by several mechanisms: e.g., replacing a disease-causing gene with a healthy copy of the gene, inactivating a disease-causing gene that is not functioning properly or introducing a new or modified gene into the body to help to treat a disease. The transferred genetic material can change how a single protein or group of proteins is produced by the cell.

The "AAV-based therapy" may be an immunotherapy. By "immunotherapy", we include the meaning of the treatment or prevention of a disorder or disease that involves the activation, enhancement, reduction, suppression, or desensitisation of the immune system. The AAV-based therapy maybe a vaccine. Preferably, the disorder or disease is an autoimmune disorder, an allergy, or a cancer.

In the context of the second and third aspects of the invention, the AAV-based therapy is contacted with albumin, for example as described above in the first aspect of the invention, prior to being administered to a subject. Thus, it will be understood that all of the relevant preferences, for example for the AAV, albumin and contacting parameters, and anti-AAV neutralising antibody, outlined in relation to the first aspect of the invention equally apply to the second and third aspects of the invention.

In an embodiment of the second or third aspects of the invention, the method further comprises administering the AAV to the subject. For example, once the AAV has been contacted with the albumin, the albumin and AAV may be administered to a subject using any amount and any route of administration effective for preventing and/or treating the disease and/or condition in question.

In the clinical setting, it may not be practical for the medical staff or other staff to administer the albumin and AAV mixture immediately, for example, following incubation of the AAV with the albumin for the set period of time at the set temperature. Therefore, it may be desirable to first pre-mix the albumin and the AAV and store the pre-mix for a longer period of time (including freezing e.g., at about at from about -15°C to about -90°C, e.g., at from about -15°C to about -25°C, e.g., at from about -70°C to about -90°C, e.g., at about -80°C or about -20°C) in order to simplify the clinical translation of the technology.

By "subject", we include the meaning of any organism to which an AAV-based therapy may be administered, e.g., for experimental, diagnostic, prophylactic, and/or therapeutic purposes. Typical subjects include any living organisms, such as animals (e.g., mammals such as mice, rats, rabbits, non-human primates, and humans). Preferably, the subject is in need of an AAV-based therapy. By "a subject in need of an AAV-based therapy", we include the meaning of individuals who would benefit from AAV-based therapy.

In a preferred embodiment, the subject is one that is known or expected to have anti-AAV neutralising antibodies to the AAV that the particular AAV-based therapy contains. Thus, it will be appreciated that the method of the second or third aspect of the invention may comprise a first step of assessing whether the subject has, or is expected to have, anti-AAV neutralising antibodies to the AAV that the particular AAV-based therapy contains, and if so, a second step of contacting the AAV with albumin prior to administering the AAV to a subject, optionally followed by a third step of administering the AAV that has been contacted with albumin to the subject. In this way, the efficacy of an AAV-based therapy for that subject is expected to be improved. Similarly, the neutralisation of the AAV-based therapy by anti-AAV neutralising antibodies is expected to be reduced. Optionally, the titre of the anti-AAV neutralising antibodies in a subject may be determined. Optionally the titre may be used to determine a suitable dose, or dose range, of the AAV-based therapy. For example a subject having a higher titre of anti-AAV neutralising antibodies may be treated with a higher dose of AAV-based therapy than a subject having a lower titre of anti-AAV neutralising antibodies. Methods for assessing whether a given subject has, or is expected to have, anti-AAV neutralising antibodies are well known in the art. Cell-based assays may be used where patient serum is mixed with AAV serotype in serial dilutions, and if the serum contains neutralising antibodies, they inactivate AAV transduction into cells and thereby reduce reporter gene expression. ELISA-based assays may be used where anti-AAV antibodies are directly detected by binding to AAV particles on a solid surface. Neutralising antibody assays as described in the Examples may be used, and total antibody assays may be used. Subjects that are expected to have anti-AAV neutralising antibodies can be identified as such by reviewing treatment history, for example if they have previously had an AAV-based treatment, they will be expected to have anti-AAV neutralising antibodies.

By "improving the efficacy of an AAV-based therapy", we include the meaning that the therapy achieves the desired clinical result using a lower dosing regimen of the AAV-based therapy. By contacting the AAV with albumin, it is believed that the neutralising effect of anti-AAV neutralising antibodies is reduced such that a higher proportion of AAV particles will successfully transduce cells. Improving the efficacy in this way may thereby reduce unwanted side effects or adverse effects. Those skilled in the art would be capable of selecting an appropriate assay to measure the desired clinical result of any given AAV-based therapy, and thereby assess the efficacy of the AAV-based therapy. ELISA assays may also be used to test the efficacy of AAV in clinical trials.

Typically, the efficacy is improved by virtue of the preservation of an activity of the AAV in the presence of an anti-AAV neutralising antibody, such as the ability to transduce or infect a cell.

By "reducing the neutralisation of an AAV-based therapy", we include the meaning of mitigating the effects of anti-AAV neutralising antibodies in a subject on the ability of the AAV-based therapy to achieve its desired clinical result. By contacting the AAV with albumin, it is believed that the neutralising effect of anti-AAV neutralising antibodies is reduced such that a higher proportion of AAV particles will successfully transduce cells, and therefore achieve the desired clinical result.

In an embodiment of the second and third aspects of the invention, the method or use enables a higher therapeutic payload to be delivered to cells per AAV dose than the corresponding therapeutic payload delivered to cells per AAV dose when the AAV is not contacted with albumin prior to administering the AAV to a subject. By 'therapeutic payload' in this context, we include the meaning of the genetic cargo that is intended to be delivered to a cell for the AAV-based therapy to mediate its therapeutic effect.

Similarly, it will be appreciated that the method or use of the second and third aspects of the invention may be useful in reducing the overall cost of an AAV-based therapy, for example by achieving the desired clinical result using a lower dosing regimen of an AAV-based therapy. In other words, the method or use may be useful in reducing the effective dose of an AAV-based therapy (e.g. reducing the number of virus particles administered to the subject and/or reducing the volume of the dose), optionally while maintaining or increasing the efficacy of the therapy. For example, the method or use of the second or third aspects of the invention may be useful in reducing the effective dose of an AAV-based therapy by at least about 2-fold, 5-fold, 10-fold, 20-fold, 30-fold, 40-fold, 50-fold, 60-fold, 70-fold, 80-fold, 90-fold and more preferably by at least about 100-fold. For example, the effective dose of an AAV-based therapy may be reduced from about an MOI of about $10^6$ to an MOI of about $10^4$.

Additionally, it will be appreciated that the method or use of the second and third aspects of the invention may be useful in reducing adverse effects (e.g. immune response and/or inflammation) associated with an AAV-based therapy. For example, activation of a patient's immune cells due to the presence of pre-existing anti-AAV neutralising antibodies that interact with the AAV-based therapy, may lead to unwanted immune response and/or inflammation.

Hence, the invention also includes:

- a method of reducing the cost of an AAV-based therapy, the method comprising contacting an AAV with albumin prior to administering the AAV to a subject;
- a use of albumin to reduce the cost of an AAV-based therapy, wherein the use involves contacting an AAV with albumin prior to administering the AAV to a subject;
- a method of reducing the effective dose of an AAV-based therapy, the method comprising contacting an AAV with albumin prior to administering the AAV to a subject;
- a use of albumin to reduce the effective dose of an AAV-based therapy, wherein the use involves contacting an AAV with albumin prior to administering the AAV to a subject;
- a method of reducing adverse effects (e.g. immune response and/or inflammation) associated with an AAV-based therapy, the method comprising contacting an AAV with albumin prior to administering the AAV to a subject; and
- a use of albumin to reduce adverse effects (e.g. immune response and/or inflammation) associated with an AAV-based therapy, wherein the use involves contacting an AAV with albumin prior to administering the AAV to a subject.

Preferences for the AAV, albumin, contact parameters and subject include those described above, for example in relation to the first aspect of the invention.

A fourth aspect of the invention provides a method of treating a disease or condition with an AAV-based therapy, the method comprising (a) contacting an AAV with albumin prior to administering the AAV to a subject; and (b) administering the AAV to the subject. As described in relation to the second and third aspects of the invention, above, the fourth aspect of the invention may include:

- assessing whether or not the subject has, or is expected to have, anti-AAV neutralising antibodies to the AAV that the particular AAV-based therapy contains, and/or

- determining the subject's titre of such anti-AAV neutralising antibodies, and/or
- using that titre to determine a suitable dose, or dose range, of the AAV-based therapy.

Similarly, the invention provides a combination of an AAV and albumin for use in treating a disease or condition with an AAV-based therapy, wherein the AAV is contacted with albumin prior to administering the AAV to a subject.

Likewise, the invention provides a use of a combination of AAV and albumin in preparing a medicament for treating a disease or condition with an AAV-based therapy, wherein the AAV is contacted with albumin prior to administering the AAV to a subject.

Preferably, the AAV and albumin are co-formulated, for example to allow the albumin to contact and coat the AAV, prior to administration to a subject. Alternatively, the AAV can be contacted with the albumin, for example for a set period of time at a set temperature as described above in relation to the first aspect of the invention, just before administering to a subject.

Preferences for the AAV, albumin, contact parameters, anti-AAV neutralising antibody and subject include those described above, for example in relation to the first, second and third aspects of the invention.

By "treatment" or "treating", we include the meaning of obtaining a therapeutically or prophylactically beneficial or desired result including and preferably a beneficial or desired clinical result. Such beneficial or desired clinical results include, but are not limited to, one or more of the following: reducing the proliferation of (or destroying) cancerous cells or other diseased tissue, decreasing symptoms resulting from a disease, increasing the quality of life of those suffering from the disease, decreasing the dose of other medications required to treat the disease, delaying the progression of the disease, and/or prolonging survival of individuals.

Preferences for the disease or condition include any of those described above in relation to the second and third aspects of the invention. The disease or condition may be any disease or condition that can be treated with an AAV-based therapy. The disease or condition may be a monogenic disorder, which includes conditions caused by mutations in a single gene such as cystic fibrosis, haemophilia and muscular dystrophy. The disease may be a neurodegenerative disease such as an age-related neurodegenerative disease like Alzheimer's disease, Parkinson's disease and amyotrophic lateral sclerosis. The disease may be a cardiovascular disease including ophthalmic diseases, blood disorders and immune system diseases. Specific examples include lipoprotein lipase deficiency, spinal muscular atrophy, aromatic L-amino acid decarboxylase (AADC) deficiency, Haemophilia B, Haemophilia A, Duchenne muscular dystrophy (DMD), Rett Syndrome and SOD1-associated amyotrophic lateral sclerosis, and the skilled person would be able to identify further diseases or conditions treatable by an AAV-based therapy.

The inventors have found that contacting AAV with albumin can protect the AAV from the effects of anti-neutralising antibodies. As outlined above, many individuals have pre-existing antibodies against AAV due to prior exposure to AAV, either wild-type AAV or previous AAV-based therapies. These antibodies can recognise and neutralise AAV vectors used in gene therapy, such that when patients with pre-existing antibodies receive AAV-based therapies, their immune response reduces treatment efficacy and/or increases the risk of adverse effects. However, by contacting the AAV with albumin prior to administering the AAV to a subject, the AAV can be protected from any anti-AAV neutralising antibodies present in the subject and still be available to treat the disease or condition to achieve the desired clinical outcome. Thus, the invention enables the treatment of subjects that have anti-AAV neutralising antibodies, which subjects may previously not have been amenable to treatment with an AAV-based therapy, or which would have been deemed inappropriate for AAV-based therapy otherwise.

Accordingly, in one embodiment, the subject is one that has anti-AAV neutralising antibodies. For example, the subject may have an anti-AAV neutralising antibody titre of at least about 1:2, 1:3, 1:4, 1:5, 1:10, 1:20, 1:30, 1:40, 1:50, 1:60, 1:70, 1:80, 1:90, 1:100, 1:200, 1:1000, 1:1100, 1:1200, 1;1300, 1;1400, 1;1500, 1;1600, 1;1700, 1;1800, 1:1900 or 1:2000. The subject may have anti-AAV neutralising antibodies that bind to one or more AAV subtypes.

Methods for assessing the presence of anti-AAV neutralising antibodies are well known in the art and include those described above in relation to the second and third aspects of the invention, such as transduction inhibition assays and total antibody assays. As with the second and third aspects of the invention, the method of the fourth aspect of the invention may include a first step of assessing whether a subject has anti-AAV neutralising antibodies, before proceeding with contacting the AAV with albumin, and administering to the subject.

In clinical trials involving AAV-based therapies, exclusion criteria relating the presence of anti-AAV neutralising antibodies are important to ensure patient safety and treatment efficacy. Patients with pre-existing neutralising antibodies above a certain threshold are generally excluded, and the particular threshold will vary from trial to trial and the AAV subtype in question. It will be appreciated therefore, that the subject may be one who would have been deemed inappropriate for AAV-based therapy for a particular disease or condition, for example because the concentration of anti-AAV neutralising antibody in the subject was above an exclusion criteria threshold for that disease or condition and AAV-based therapy. In this way, the technology of the present invention may be used to enable more subjects to benefit from AAV-based therapies that would otherwise be the case, and so assist with patient recruitment in clinical trials where the trial therapy is an AAV-based therapy. A skilled person will be able to identify an exclusion criteria threshold for a given AAV subtype, and for a particular disease or condition, as a matter of routine. For example, by assessing the transgene expression in the presence of a serial dilution of antibody titres, the concentration of antibody that neutralises the effect the AAV can be determined (e.g. one that abolishes transgene expression or reduces it to below a set level relative to the transgene expression in the absence of neutralising antibody) and a threshold defined accordingly.

In one clinical trial (NCT02926066), an exclusion threshold of a titre greater than 1:1200 was applied for an AAV2-based therapy. Thus, the subject may be one that has a titre of anti-AAV2 neutralising antibody of greater than 1:1200, for example when the AAV-based therapy is an AAV2-based therapy.

In another clinical trial (NCT04783181), an exclusion threshold of any anti-AAV5 neutralising antibody was applied. Thus, the subject may be one that has any anti-AAV5 neutralising antibody, for example when the AAV-based therapy is an AAV5-based therapy.

In one clinical trial (NCT04394286), an exclusion threshold of a titre over 1:5 was applied for an AAV8-based therapy. Thus, the subject may be one that has a titre of anti-AAV8 neutralising antibody of greater than 1:5, for example when the AAV-based therapy is an AAV8-based therapy.

In one clinical trial (NCT05832684), an exclusion threshold of a titre greater than or equal to 1:1000 was applied for an rAAV8-based therapy. Thus, the subject may be one that has a titre of anti-rAAV8 neutralising antibody of greater than or equal to 1:1000, for example when the AAV-based therapy is an rAAV8-based therapy.

In another embodiment, the subject is one that does not have anti-AAV neutralising antibodies, for example anti-AAV neutralising antibodies against one or more AAV subtypes. While such subjects do not have pre-existing anti-AAV neutralising antibodies that would neutralise a first AAV-based therapy, it is expected that by contacting the AAV with albumin, the subject will not generate (or will generate at a lower level) anti-AAV neutralising antibodies to the first AAV-based therapy, and so will be able to benefit from a second or subsequent AAV-based therapy. In other words, when the subject is one that does not have anti-AAV neutralising antibodies, and is being administered a first AAV-based therapy, it will be appreciated that the method may be used to improve the efficacy of a second or subsequent AAV-based therapy.

The use of the word "a" or "an" when used in conjunction with the term "comprising" in the claims and/or the specification may mean "one," but it is also consistent with the meaning of "one or more," "at least one," and "one or more than one."

When we refer to a range, for example a temperature range of "0 to 35°C", we include the meaning of about or approximately 0°C to about or approximately 35°C.

The listing or discussion in this specification of an apparently prior-published document should not necessarily be taken as an acknowledgement that the document is part of the state of the art or is common general knowledge.

Preferred, non-limiting examples which embody certain aspects of the invention will now be described, with reference to the figures.

## FIGURES

FIGURE 1: Effect of nAb on the transduction of AAV2 into HeLa cells. * indicates a statisically significant difference ($p \leq 0.011$).

FIGURE 2: Effect of nAb on the transduction of (a) AAV5 into Huh7 D12 cells, * indicates a statisically significant difference ($p \leq 0.0563$), (b) AAV8 into HEK293 cells, all samples are statistically significantly different ($p \leq 0.0104$), (c) AAV6 into HMEC cells * indicates a statisically significant difference ($p \leq 0.0475$).

FIGURE 3: Plate layout for nAb assay.

FIGURE 4: Effect of precoating AAV2 with different concentrations of Saccharomyces derived recombinant albumin on the transduction of AAV8 into HEK293 cells. The highest dilution of nAb (1:7290) is shown and the percentage transduction that was achieved for AAV8 either in the presence or absence of albumin. * indicates a statisically significant difference ($p \leq 0.027$).

## EXAMPLES

## Results and Discussion

In order to establish if the pre-treatment of AAV with albumin conferred a protective effect against the action of neutralising anti-AAV antibody (nAb), AAV2 (containing a GFP gene) was preincubated with a Saccharomyces derived albumin (200mg/mL albumin, 145 mM sodium, 16 mM octanoate, 50 μg/mL Polysorbate 80 (i.e. trace level of Polysorbate 80)) for 2hrs at 4°C before being added to HeLa cells. As a control, AAV2 was also preincubated with buffer (sodium 145mM, octanoate 16mM, Polysorbate trace) for 2 hrs at 4°C before addition to the cells. After the pre incubation period, the AAV2 was added to a plate containing serum-free medium in the presence and absence of a serial dilution of neutralising antibody and allowed to incubate at 37°C to enable the nAb to bind to the virus. AAV2 + buffer and AAV2 preincubated with Saccharomyces derived albumin were included as controls to determine the percentage of GFP expression in the absence of nAb. Once the incubation period was completed the virus+nAb and controls were transferred to the wells of a plate containing HeLa cells and incubated at 37°C for 4 hours. After 4 hours the media was removed from the wells and replaced with serum-containing media. The cells were incubated for 48 hrs and then examined to determine the % of GFP containing cells. If the albumin was having a protective effect on the AAV2 the % of GFP-expressing cells would be expected to be higher in the samples containing Saccharomyces derived albumin compared to the samples containing buffer (sodium 145mM, octanoate 16mM, Polysorbate trace). As albumin can sometimes have a positive impact on the transduction of AAV into cells the samples were normalised against AAV2 control samples which were not exposed to any nAb.

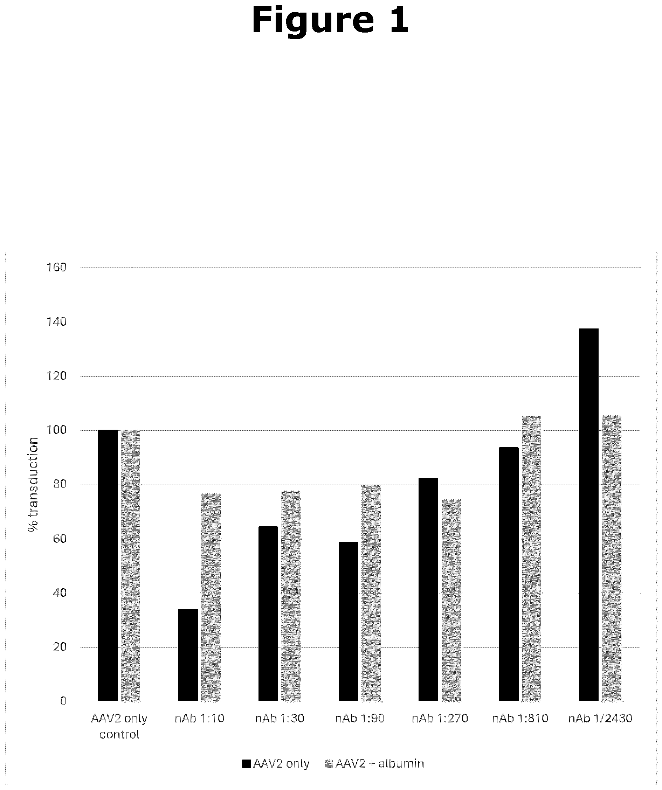

As can be seen from Figure 1, the AAV2 incubated in the presence of albumin ("AAV2 + albumin") was less inhibited by the nAb than the AAV2 incubated with buffer ("AAV2 only"). In the presence of albumin, the AAV2 was able to transduce cells approximately 80% of cells for dilutions 1:10, 1:30 and 1:90 compared to 34%, 64% and 58% transduction for the AAV2 with buffer. This indicates that the albumin was able to protect the AAV2 against nAb whereas when no albumin was present the nAbs were able to bind to the AAV2 and prevent it from transducing the cells. When the nAb was diluted 1:270 or lower no impact on transduction levels were observed for samples with or without albumin present indicating that the nAbs were too dilute to have an impact on the transduction.

Effect of Different Cell Lines and AAV Serotypes on Protection from nAb

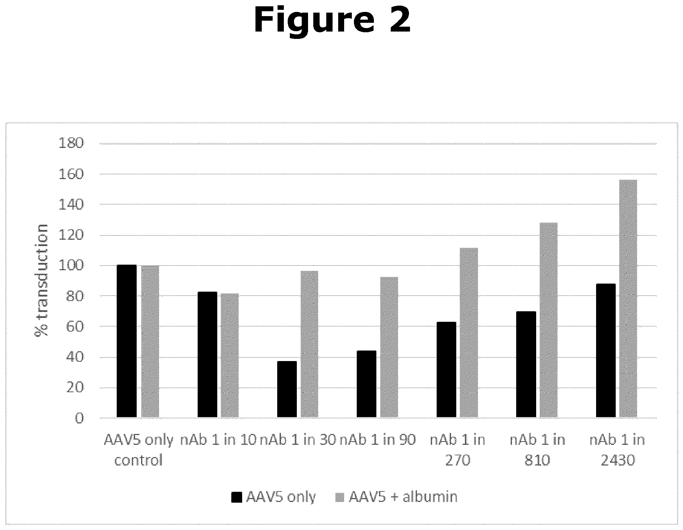

In order to determine the protective effect against nAb by pre incubating the AAV with albumin, a number of different cell lines and AAV serotypes were examined. Figure 2a shows the effect of preincubating AAV5 (containing the GFP gene) with a Saccharomyces derived albumin (200 mg/mL) before exposure to anti-AAV5 antibodies and subsequent transduction into Huh7 D12 cells. As can be seen from Figure 2a the albumin had a protective effect on the AAV5. When AAV5 was preincubated with the albumin the % transduction was reduced to 81% when the nAb was diluted 1:10 and as the nAb became more dilute the % transduction was restored to the level seen or higher than the no nAb control. This demonstrates that the precoating of AAV5 with albumin is conferring protection against the nAb. Conversely, for the AAV5 with buffer control the % transduction showed a linear increase as the nAb became more dilute from 1:30 dilution upto 1:2430 dilution. Also, at the lowest dilution of nAb (1:2430) the % transduction was not restored to the level seen for the no nAb control indicating that the nAb would need to be further diluted to no longer inhibit the AAV5 transduction into Huh7 D12 cells.

In Figure 2b the protective effects of albumin against anti-AAV8 antibodies can be seen. Again, the AAV8 (containing the luciferase gene) was preincubated with a Saccharomyces derived albumin (200 mg/mL, sodium 145mM, octanoate 16mM, Polysorbate trace) before exposure to anti-AAV8 antibodies and transduction into HEK293 cells. In this example the albumin was very effective at protecting the AAV8 from the nAbs. At a nAb dilution of 1:90 the percentage transduction was restored to 75% of the control for the albumin coated AAV8 compared to 2.8% transduction for the non albumin coated AAV8 sample. In addition, for the albumin coated AAV8 transduction was restored to that of the control for the 1:7290 dilution whereas the same dilution without albumin precoating was 6.91%.

This nAb protective effect was also observed with HMEC cells and AAV6 (containing a GFP gene). Figure 2c illustrates that at a 1:2000 dilution of nAb both the albumin coated and non coated AAV6 gave similar % transduction levels. However, as the nAbs are more diluted the % transduction increased more for the precoated AAV6 compared to the non coated AAV6. This demonstrates that the technique of precoating the AAV with a Saccharomyces derived albumin is applicable to various cell lines and AAV serotypes

Effect of Concentration of Albumin on protection against nAb

To establish the concentration range of Saccharomyces derived albumin that provides the protective effect against nAb a range on albumin concentrations were examined. Saccharomyces derived albumin was used either undiluted (200mg/mL albumin, sodium 145mM, octanoate 16mM, Polysorbate trace) or diluted in buffer (sodium 145mM, octanoate 16mM, Polysorbate trace) to 100mg/mL, 50mg/mL or 20mg/mL. For this work AAV8 expressing luciferase was used in transductions into HEK293 cells. In Figure 4 it can be seen that at the highest dilution of AAV8 nAb there was a marked difference in the levels of transduction observed. The percentage transduced cells increases from 3.05 to 10.31 to 19.31 to 21.8% for 20, 50, 100 and 200mg/mL albumin respectively. Whereas the maximum percentage transduction seen for non precoated AAV8 was 5.6%. This indicates that albumin concentrations >20mg/mL provide protection to AAV against nAb

**Materials and Methods**

Cell Lines and AAV

The HEK293, HeLa and Huh7-D12 cells were provided by the European Collection of Authenticated Cell Cultures (ECACC). The HMEC cells were provided by the Centre for Disease Control (CDC, USA). HEK293 and HeLa cells were grown in MEM basal media (Gibco, UK) supplemented with 10 % FBS (Gibco, USA), 2mM L-glutamine (Gibco, UK), 1% non-essential amino acids (Gibco, UK) and 1 % penicillin/streptomycin (Gibco, USA). Huh7-D12 cells were cultured in DMEM high glucose basal media (Gibco, UK) supplemented with 10 % FBS (Gibco, USA) and 2mM L-glutamine (Gibco, UK). HMEC cells were cultured in MCDB131 media (Gibco, USA) supplemented with 10% FBS (Gibco, USA), 2mM L-glutamine (Gibco, UK), 1ug/mL hydrocortisone (StemCell Technologies, UK) and 10ng/mL recombinant human epidermal growth factor (Peprotech, UK). All cells were maintained at 37°C, 5% $CO_2$ in a humidified atmosphere. Control AAV particles were supplied by Charles River (USA) and were comprised of either the luciferase gene (Luc) or the gene for green fluorescent protein (GFP) under the control of the Cytomegalovirus (CMV) promoter.

Sources of Albumin

Saccharomyces derived human albumin (200mg/mL albumin sodium 145mM, octanoate 16mM, Polysorbate trace).
All albumins tested were mature sequence wild-type human albumin (SEQ ID NO: 2).

Concentrations of Albumin

In general, 200mg/ml Saccharomyces derived albumin was utilised in buffer (sodium 145mM, octanoate 16mM, polysorbate trace). When required, Saccharomyces derived albumin was diluted to 100mg/ml, 50mg/ml and 20mg/ml in buffer (sodium 145mM, octanoate 16mM, polysorbate trace).

Flow Cytometry Analysis for GFP expression

Following transduction, adherent cells were removed from the wells by trypsin (Gibco, USA), washed and resuspended in BD Pharmingen Stain Buffer (BD, USA). Flow cytometry analysis 10,000 events were analysed for forward and side scatter characteristics and GFP light emission using the FITC filter set (530/30) on a BD Celesta (BD, USA). The percentage of transduced cells was determined by comparing the GFP-expressing transduced cells to non-transduced controls using FlowJo software (Becton Dickinson, USA).

Luciferase assay

The luciferase assay was performed utilising the Bright-Glo luciferase assay system (Promega, UK) following the manufacturer's instructions. The cells to be analysed were removed from the $CO_2$ incubator and left for 15 minutes to equilibrate to room temperature. A 100μl aliquot Bright-Glo™ Reagent was added to the wells of the plate and the plate was incubated at room temperature for 3 minutes to allow the cells to lyse. The plate was then transferred to an Enspire (Perkin Elmer, UK) plate reader to measure the levels of bioluminescence.

Neutralising Antibody Assay

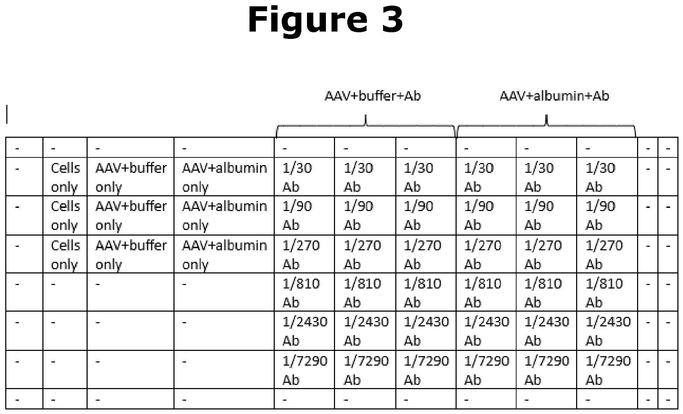

Anti-AAV antibodies were supplied from several sources. Anti-AAV2, AAV5 and AAV6 were supplied by Origene (USA). Anti-AAV2, AAV5, AAV6 and AAV8 were also supplied from Progen (Germany). For the nAb assay cells were cultured and seeded into a 96 well plate at $1 \times 10^3$ cells/well (100μl media was used per well) and incubated overnight. Various AAV MOI's were tested (MOI $10^2$-$10^6$) during the experiments. For albumin-containing samples the required amount of AAV was added to a 16μl aliquot of albumin (200mg/mL) (AAV+albumin), briefly vortexed and incubated at 4°C for 2 hours. For virus-only controls the same volume of AAV was added to buffer (sodium 145mM, octanoate 16mM, polysorbate trace) (AAV+buffer), briefly vortexed and incubated at 4°C for 2 hours. Anti-AAV antibody dilutions were performed in a 96 well U-bottom plate. In general, the following dilution series was utilised, illustrated in Figure 3, however, depending on the cell type/AAV serotype differing dilution series were sometimes employed. One hundred microlitres of serum-free media was added to each well, other than the wells labelled 1/30 Ab, to these wells 145μl of serum-free media was added. Five microliters of anti-AAV antibody was then added to the 1/30 Ab wells resulting in a 1/30 dilution of the anti-AAV antibody. Fifty microlitres of this 1/30 dilution was then taken from the 1/30 well and added and mixed to the 1/90 Ab well, this procedure was repeated down the plate in the 1/270 Ab, 1/810 Ab, 1/2430 Ab and 1/7290 Ab wells. When the final dilution was completed 50μl of diluted anti-AAV antibody was removed and discarded from the 1/7290 Ab wells so that all the wells contained 100μl. Once the AAV had been incubated in the albumin or buffer 16μl was added to the 96 well U-bottom plate containing the antibody dilution series. The AAV+buffer, AAV+albumin and anti-AAV antibodies were incubated at 37°C for 1 hour. Cells only, AAV+buffer only and AAV+albumin only controls were also included in the plate (see Figure 3 where "-" indicates an empty well). After the incubation period, the media was removed from the cells and replaced with the serum-free media from the 96 well U-bottom plate containing the AAV+buffer, AAV+albumin and anti-AAV antibodies. The cells were incubated for 4 hours at 37°C, 5% $CO_2$ after which the media was removed and replaced with fresh serum-containing media. The cells were incubated for a further 48 hours and then examined for the presence of Luc- or GFP-expressing cells.

**References**

Calcedo, R., & Wilson, J. M. (2013). Humoral Immune Response to AAV. Frontiers in Immunology, 4. https://doi.org/10.3389/fimmu.2013.00341

Earley, J., Piletska, E., Ronzitti, G., & Piletsky, S. (2023). Evading and overcoming AAV neutralization in gene therapy. In Trends in Biotechnology (Vol. 41, Issue 6, pp. 836-845). Elsevier Ltd. https://doi.org/10.1016/j.tibtech.2022.11.006

Francis, G. L. (2010). Albumin and mammalian cell culture: Implications for biotechnology applications. In Cytotechnology (Vol. 62, Issue 1, pp. 1-16). https://doi.org/10.1007/s10616-010-9263-3

Gross, D. A., Tedesco, N., Leborgne, C., & Ronzitti, G. (2022). Overcoming the Challenges Imposed by Humoral Immunity to AAV Vectors to Achieve Safe and Efficient Gene Transfer in Seropositive Patients. In Frontiers in Immunology (Vol. 13). Frontiers Media S.A. https://doi.org/10.3389/fimmu.2022.857276

Issa, S. S., Shaimardanova, A. A., Solovyeva, V. V., & Rizvanov, A. A. (2023). Various AAV Serotypes and Their Applications in Gene Therapy: An Overview. In Cells (Vol. 12, Issue 5). MDPI. https://doi.org/10.3390/cells12050785

Laughlin, C. A., Cardellichio, C. B., & Coon, H. C. (1986). Latent Infection of KB Cells with Adeno-Associated Virus Type 2. In JOURNAL OF VIROLOGY (Vol. 60, Issue 2).

Mariam, J., Sivakami, S., & Dongre, P. M. (2016). Albumin corona on nanoparticles-a strategic approach in drug delivery. In Drug Delivery (Vol. 23, Issue 8, pp. 2668-2676). Taylor and Francis Ltd. https://doi.org/10.3109/10717544.2015.1048488

Matsushita, T., Elliger, S., Elliger, C., Podsakoff, G., Villarreal, L., Kurtzman, G. J., Iwaki, Y., & Colosi, P. (1998). Adeno-associated virus vectors can be efficiently produced without helper virus. In Gene Therapy (Vol. 5). http://www.stockton-press.co.uk/gt

Mendell, J. R., Connolly, A. M., Lehman, K. J., Griffin, D. A., Khan, S. Z., Dharia, S. D., Quintana-Gallardo, L., & Rodino-Klapac, L. R. (2022). Testing preexisting antibodies prior to AAV gene transfer therapy: rationale, lessons and future considerations. In Molecular Therapy Methods and Clinical Development (Vol. 25, pp. 74-83). Cell Press. https://doi.org/10.1016/j.omtm.2022.02.011

Sand, K. M. K., Bern, M., Nilsen, J., Dalhus, B., Gunnarsen, K. S., Cameron, J., Grevys, A., Bunting, K., Sandlie, I., & Andersen, J. T. (2014). Interaction with Both Domain I and III of Albumin Is Required for Optimal pH-dependent Binding to the Neonatal Fc Receptor (FcRn) *. Journal of Biological Chemistry, 289(50), 34583-34594. https://doi.org/10.1074/j bc. M 114.587675

Sarkar, S. N., Corbin, D., & Simpkins, J. W. (2024). Brain-Wide Transgene Expression in Mice by Systemic Injection of Genetically Engineered Exosomes: CAP-Exosomes. Pharmaceuticals, 17(3). https://doi.org/10.3390/ph17030270

Wang, J. H., Gessler, D. J., Zhan, W., Gallagher, T. L., & Gao, G. (2024). Adeno-associated virus as a delivery vector for gene therapy of human diseases. In Signal Transduction and Targeted Therapy (Vol. 9, Issue 1). Springer Nature. https://doi.org/10.1038/s41392-024-01780-w

Wang, M., Sun, J., Crosby, A., Woodard, K., Hirsch, M. L., Samulski, R. J., & Li, C. (2017). Direct interaction of human serum proteins with AAV virions to enhance AAV transduction: Immediate impact on clinical applications. Gene Therapy, 24(1), 49-59. https://doi.org/10.1038/gt.2016.75

List of sequences described herein

>SEQ ID NO: 1

gacgctcacaagtccgaagtcgctcacagattcaaggacttgggtgaagaaaacttcaaggctttggtcttgatcgctttc
gctcaatacttgcaacaatgtccattcgaagatcacgtcaagttggtcaacgaagttaccgaattcgctaagacttgtgtt
gctgacgaatccgcggaaaactgtgacaagtccttgcacaccttgttcggtgataagttgtgtactgttgctaccttgaga
gaaacctacggtgaaatggctgactgttgtgctaagcaagaaccagaaagaaacgaatgtttcttgcaacacaaggac
gacaacccaaacttgccaagattggttagaccagaagttgacgtcatgtgtactgctttccacgacaacgagaaacctt
cttgaagaagtacttgtacgaaattgctagaagacacccatacttctacgctccagaattgttgttcttcgctaagagatac
aaggctgctttcaccgaatgttgtcaagctgctgataaggctgcttgtttgttgccaaagttggatgaattgagagacgaa
ggtaaggctagctccgcaaagcaaagattgaagtgtgcttccttgcaaaagttcggtgaaagagctttcaaggcttggg
ctgtcgctagattgtctcaaagattcccaaaggctgaattcgctgaagtttctaagttggttactgacttgactaaggttcac
actgaatgttgtcacggtgacttgttggaatgtgctgatgacagagctgacttggctaagtacatctgtgaaaaccaaga
ctctatctcttccaagttgaaggaatgttgtgaaaagccattgttggaaaagtctcactgtattgctgaagttgaaaacgat
gaaatgccagctgacttgccatctttggctgctgacttcgttgaatctaaggacgtttgtaagaactacgctgaagctaag
gacgtcttcttgggtatgttcttgtacgaatacgctagaagacacccagactactccgttgtcttgttgttgagattggctaa
gacctacgaaactacccctcgagaagtgttgtgctgctgctgacccacacgaatgttacgctaaggttttcgatgaattcaa
gccattggtcgaagaaccacaaaacttgatcaagcaaaactgtgaattgttcgaacaattgggtgaatacaagttccaa
aacgctttgttggttagatacactaagaaggtcccacaagtctccaccccaactttggttgaagtctctagaaacttgggta
aggtcggttctaagtgttgtaagcacccagaagctaagagaatgccatgtgctgaagattacttgtccgtcgttttgaacc
aattgtgtgttttgcacgaaaagaccccagtctctgatagagtcaccaagtgttgtactgaatctttggttaacagaagac
catgtttctctgctttggaagtcgacgaaacttacgttccaaaggaattcaacgctgaaactttcaccttccacgctgatatc
tgtaccttgtccgaaaaggaaagacaaattaagaagcaaactgctttggttgaattggtcaagcacaagccaaaggcta
ctaaggaacaattgaaggctgtcatggatgatttcgctgctttcgttgaaaagtgttgtaaggctgatgataaggaaactt
gtttcgctgaagaaggtaagaagttggtcgctgcttcccaagctgccttaggtttgtaataa

>SEQ ID NO: 2

DAHKSEVAHRFKDLGEENFKALVLIAFAQYLQQCPFEDHVKLVNEVTEFAKTCVADESAENCDK
SLHTLFGDKLCTVATLRETYGEMADCCAKQEPERNECFLQHKDDNPNLPRLVRPEVDVMCTAFH

DNEETFLKKYLYEIARRHPYFYAPELLFFAKRYKAAFTECCQAADKAACLLPKLDELRDEGKASSA
KQRLKCASLQKFGERAFKAWAVARLSQRFPKAEFAEVSKLVTDLTKVHTECCHGDLLECADDRA
DLAKYICENQDSISSKLKECCEKPLLEKSHCIAEVENDEMPADLPSLAADFVESKDVCKNYAEAK
DVFLGMFLYEYARRHPDYSVVLLLRLAKTYETTLEKCCAAADPHECYAKVFDEFKPLVEEPQNLIK
QNCELFEQLGEYKFQNALLVRYTKKVPQVSTPTLVEVSRNLGKVGSKCCKHPEAKRMPCAEDYL
SVVLNQLCVLHEKTPVSDRVTKCCTESLVNRRPCFSALEVDETYVPKEFNAETFTFHADICTLSEK
ERQIKKQTALVELVKHKPKATKEQLKAVMDDFAAFVEKCCKADDKETCFAEEGKKLVAASQAAL
GL

>SEQ ID NO: 3

MKWVTFLLLLFVSGSAFSRGVFRREAHKSEIAHRYNDLGEQHFKGLVLIAFSQYLQKCSYDEHA
KLVQEVTDFAKTCVADESAANCDKSLHTLFGDKLCAIPNLRENYGELADCCTKQEPERNECFLQ
HKDDNPSLPPFERPEAEAMCTSFKENPTTFMGHYLHEVARRHPYFYAPELLYYAEQYNEILTQCC
AEADKESCLTPKLDGVKEKALVSSVRQRMKCSSMQKFGERAFKAWAVARLSQTFPNADFAEIT
KLATDLTKVNKECCHGDLLECADDRAELAKYMCENQATISSKLQTCCDKPLLKKAHCLSEVEHD
TMPADLPAIAADFVEDQEVCKNYAEAKDVFLGTFLYEYSRRHPDYSVSLLLRLAKKYEATLEKCC
AEANPPACYGTVLAEFQPLVEEPKNLVKTNCDLYEKLGEYGFQNAILVRYTQKAPQVSTPTLVEA
ARNLGRVGTKCCTLPEDQRLPCVEDYLSAILNRVCLLHEKTPVSEHVTKCCSGSLVERRPCFSAL
TVDETYVPKEFKAETFTFHSDICTLPEKEKQIKKQTALAELVKHKPKATAEQLKTVMDDFAQFLD
TCCKAADKDTCFSTEGPNLVTRCKDALA

>SEQ ID NO: 4

MKWVTFLLLLFISGSAFSRGVFRREAHKSEIAHRFKDLGEQHFKGLVLIAFSQYLQKCPYEEHIKL
VQEVTDFAKTCVADENAENCDKSIHTLFGDKLCAIPKLRDNYGELADCCAKQEPERNECFLQHK
DDNPNLPPFQRPEAEAMCTSFQENPTSFLGHYLHEVARRHPYFYAPELLYYAEKYNEVLTQCCTE
SDKAACLTPKLDAVKEKALVAAVRQRMKCSSMQRFGERAFKAWAVARMSQRFPNAEFAEITKL
ATDVTKINKECCHGDLLECADDRAELAKYMCENQATISSKLQACCDKPVLQKSQCLAEIEHDNI
PADLPSIAADFVEDKEVCKNYAEAKDVFLGTFLYEYSRRHPDYSVSLLLRLAKKYEATLEKCCAEG
DPPACYGTVLAEFQPLVEEPKNLVKTNCELYEKLGEYGFQNAVLVRYTQKAPQVSTPTLVEAARN
LGRVGTKCCTLPEAQRLPCVEDYLSAILNRLCVLHEKTPVSEKVTKCCSGSLVERRPCFSALTVD
ETYVPKEFKAETFTFHSDICTLPDKEKQIKKQTALAELVKHKPKATEDQLKTVMGDFAQFVDKCC
KAADKDNCFATEGPNLVARSKEALA

>SEQ ID NO: 5

MKWVTFISLLFLFSSAYSRGVFRRDTHKSEVAHRFKDLGEEHFKGLVLVAFSQYLQQCPFEEHV
KLVNEVTEFAKTCVADESAENCDKSLHTLFGDKLCTVATLRETYGEMADCCAKQEPERNECFLQ
HKDDNPNLPPLVRPEVDVMCTAFHDNEATFLKKYLYEVARRHPYFYAPELLFFAARYKAAFAECC
QAADKAACLLPKLDELRDEGKASSAKQRLKCASLQKFGDRAFKAWAVARLSQKFPKAEFAEVS
KLVTDLTKVHTECCHGDLLECADDRADLAKYMCENQDSISSKLKECCDKPLLEKSHCLAEVEND
EMPADLPSLAADYVESKDVCKNYAEAKDVFLGMFLYEYARRHPDYSVMLLLRLAKAYEATLEKCC
AAADPHECYAKVFDEFQPLVEEPQNLVKQNCELFEQLGEYKFQNALLVRYTKKVPQVSTPTLVEV

SRNLGKVGAKCCKLPEAKRMPCAEDYLSVVLNRLCVLHEKTPVSEKVTKCCTESLVNRRPCFSA
LELDEAYVPKAFNAETFTFHADMCTLSEKEKQVKKQTALVELVKHKPKATKEQLKGVMDNFAAF
VEKCCKADDKEACFAEEGPKFVAASQAALA

>SEQ ID NO: 6

MKWVTFISLLLLLFSSAYSRGVFRRDTHKSEIAHRFKDLGEEHFKGLVLIAFSQYLQQCPFDEHVK
LVNELTEFAKTCVADESHAGCEKSLHTLFGDELCKVASLRETYGDMADCCEKQEPERNECFLSH
KDDSPDLPKLKPDPNTLCDEFKADEKKFWGKYLYEIARRHPYFYAPELLYYANKYNGVFQECCQA
EDKGACLLPKIETMREKVLASSARQRLRCASIQKFGERALKAWSVARLSQKFPKAEFVEVTKLVT
DLTKVHKECCHGDLLECADDRADLAKYICDNQDTISSKLKECCDKPLLEKSHCIAEVEKDAIPEN
LPPLTADFAEDKDVCKNYQEAKDAFLGSFLYEYSRRHPEYAVSVLLRLAKEYEATLEECCAKDDP
HACYSTVFDKLKHLVDEPQNLIKQNCDQFEKLGEYGFQNALIVRYTRKVPQVSTPTLVEVSRSLG
KVGTRCCTKPESERMPCTEDYLSLILNRLCVLHEKTPVSEKVTKCCTESLVNRRPCFSALTPDETY
VPKAFDEKLFTFHADICTLPDTEKQIKKQTALVELLKHKPKATEEQLKTVMENFVAFVDKCCAAD
DKEACFAVEGPKLVVSTQTALA

>SEQ ID NO: 7

MKWVTFISLLFLFSSAYSRGVFRRDTYKSEIAHRFKDLGEQYFKGLVLIAFSQHLQQCPYEEHVK
LVREVTEFAKTCVADESAENCDKSIHTLFGDKLCAIPSLREHYGDLADCCEKEEPERNECFLQHK
NDNPDIPKLKPDPVALCADFQEDEQKFWGKYLYEIARRHPYFYAPELLYYAIIYKDVFSECCQAA
DKAACLLPKIEHLREKVLTSAAKQRLKCASIQKFGERAFKAWSLARLSQRFPKADFTEISKIVTDL
AKVHKECCHGDLLECADDRADLAKYICENQDTISTKLKECCDKPLLEKSHCIAEAKRDELPADLN
PLEHDFVEDKEVCKNYKEAKHVFLGTFLYEYSRRHPDYSVSLLLRIAKIYEATLEDCCAKEDPPAC
YATVFDKFQPLVDEPKNLIKQNCELFEKLGEYGFQNALIVRYTKKVPQVSTPTLVEVARKLGLVGS
RCCKRPEEERLSCAEDYLSLVLNRLCVLHEKTPVSEKVTKCCTESLVNRRPCFSALTPDETYKPKE
FVEGTFTFHADLCTLPEDEKQIKKQTALVELLKHKPHATEEQLRTVLGNFAAFVQKCCAAPDHEA
CFAVEGPKFVIEIRGILA

>SEQ ID NO: 8

MKWVTFVSLLFLFSSAYSRGVLRRDTHKSEIAHRFNDLGEKHFKGLVLVAFSQYLQQCPFEDHV
KLVNEVTEFAKKCAADESAENCDKSLHTLFGDKLCTVATLRATYGELADCCEKQEPERNECFLTH
KDDHPNLPKLKPEPDAQCAAFQEDPDKFLGKYLYEVARRHPYFGPELLFHAEEYKADFTECCPA
DDKLACLIPKLDALKERILLSSAKERLKCSSFQNFGERAVKAWSVARLSQKFPKADFAEVSKIVT
DLTKVHKECCHGDLLECADDRADLAKYICEHQDSISGKLKACCDKPLLQKSHCIAEVKEDDLPS
DLPALAADFAEDKEICKHYKDAKDVFLGTFLYEYSRRHPDYSVSLLLRIAKTYEATLEKCCAEADP
PACYRTVFDQFTPLVEEPKSLVKKNCDLFEEVGEYDFQNALIVRYTKKAPQVSTPTLVEIGRTLGK
VGSRCCKLPESERLPCSENHLALALNRLCVLHEKTPVSEKITKCCTDSLAERRPCFSALELDEGYV
PKEFKAETFTFHADICTLPEDEKQIKKQSALAELVKHKPKATKEQLKTVLGNFSAFVAKCCGRED
KEACFAEEGPKLVASSQLALA

>SEQ ID NO: 9

MKWVTFISLLFLFSSAYSRGVFRREAHKSEIAHRFNDVGEEHFIGLVLITFSQYLQKCPYEEHAKL
VKEVTDLAKACVADESAANCDKSLHDIFGDKICALPSLRDTYGDVADCCEKKEPERNECFLHHK
DDKPDLPPFARPEADVLCKAFHDDEKAFFGHYLYEVARRHPYFYAPELLYYAQKYKAILTECCEAA
DKGACLTPKLDALEGKSLISAAQERLRCASIQKFGDRAYKAWALVRLSQRFPKADFTDISKIVTD
LTKVHKECCHGDLLECADDRADLAKYMCEHQETISSHLKECCDKPILEKAHCIYGLHNDETPAG
LPAVAEEFVEDKDVCKNYEEAKDLFLGKFLYEYSRRHPDYSVVLLLRLGKAYEATLKKCCATDDP
HACYAKVLDEFQPLVDEPKNLVKQNCELYEQLGDYNFQNALLVRYTKKVPQVSTPTLVEISRSLG
KVGSKCCKHPEAERLPCVEDYLSVVLNRLCVLHEKTPVSEKVTKCCSESLVDRRPCFSALGPDET
YVPKEFNAETFTFHADICTLPETERKIKKQTALVELVKHKPHATNDQLKTVVGEFTALLDKCCSAE
DKEACFAVEGPKLVESSKATLG

>**SEQ ID NO: 10**

MKWVTFISLLFLFSSAYSRGVFRRDAHKSEVAHRFKDLGEENFKALVLIAFAQYLQQCPFEDHVK
LVNEVTEFAKTCVADESAENCDKSLHTLFGDKLCTVATLRETYGEMADCCAKQEPERNECFLQH
KDDNPNLPRLVRPEVDVMCTAFHDNEETFLKKYLYEIARRHPYFYAPELLFFAKRYKAAFTECCQA
ADKAACLLPKLDELRDEGKASSAKQRLKCASLQKFGERAFKAWAVARLSQRFPKAEFAEVSKLV
TDLTKVHTECCHGDLLECADDRADLAKYICENQDSISSKLKECCEKPLLEKSHCIAEVENDEMPA
DLPSLAADFVESKDVCKNYAEAKDVFLGMFLYEYARRHPDYSVVLLLRLAKTYETTLEKCCAAAD
PHECYAKVFDEFKPLVEEPQNLIKQNCELFEQLGEYKFQNALLVRYTKKVPQVSTPTLVEVSRNL
GKVGSKCCKHPEAKRMPCAEDYLSVVLNQLCVLHEKTPVSDRVTKCCTESLVNRRPCFSALEVD
ETYVPKEFNAETFTFHADICTLSEKERQIKKQTALVELVKHKPKATKEQLKAVMDDFAAFVEKCC
KADDKETCFAEEGKKLVAASQAALGL

>**SEQ ID NO: 11**

MGSIGAASMEFCFDVFKELKVHHANENIFYCPIAIMSALAMVYLGAKDSTRTQINKVVRFDKLPG
FGDSIEAQCGTSVNVHSSLRDILNQITKPNDVYSFSLASRLYAEERYPILPEYLQCVKELYRGGLE
PINFQTAADQARELINSWVESQTNGIIRNVLQPSSVDSQTAMVLVNAIVFKGLWEKAFKDEDTQ
AMPFRVTEQESKPVQMMYQIGLFRVASMASEKMKILELPFASGTMSMLVLLPDEVSGLEQLESII
NFEKLTEWTSSNVMEERKIKVYLPRMKMEEKYNLTSVLMAMGITDVFSSSANLSGISSAESLKIS
QAVHAAHAEINEAGREVVGSAEAGVDAASVSEEFRADHPFLFCIKHIATNAVLFFGRCVSP

## EMBODIMENTS OF INVENTION

The invention includes the embodiments of the numbered paragraphs below.

1. A method of preserving the activity of an adeno-associated virus (AAV) in the presence of an anti-AAV neutralising antibody, the method comprising contacting the AAV with albumin.

2. Use of albumin to preserve the activity of an adeno-associated virus (AAV) in the presence of an anti-AAV neutralising antibody, wherein the use involves contacting the AAV with albumin.

3. The method according to Paragraph1 or a use according to Paragraph 2, wherein the activity of the AAV is the ability of the AAV to transduce a cell, or the ability of the AAV to infect a cell.

4. The method or use according to Paragraph 3, wherein the level of transduction of the AAV into the cell when the AAV is contacted with albumin is higher than the level of transduction of the AAV into the cell when the AAV is not contacted with albumin.

5. The method or use according to Paragraph 3 or 4, wherein the ability of the AAV to transduce a cell is assessed by a fluorescence-based method, bioluminescence, an enzyme-based method, DNA staining, polymerase chain reaction (PCR), enzyme-linked immunosorbent assay (ELISA) or western blotting.

6. The method or use according to any one of Paragraphs 3-5, wherein the cell is a dividing cell or a non-dividing cell.

7. The method or use according to any one of Paragraphs 3-6, wherein the cell is a eukaryotic cell.

8. The method or use according to any one of Paragraphs 3-7, wherein the cell is an animal cell.

9. The method or use according to Paragraph 8 wherein the animal cell is a mammalian cell, a fish cell, an insect cell, a reptile cell, an amphibian cell or an avian cell.

10. A method or use according to any one of Paragraphs 3-9, wherein the cell is any one of an epithelial cell (e.g. a nasal epithelial cell), a liver cell, a muscle cell, a heart cell, an immune cell, a pancreatic cell, an endothelial cell, a stem cell (a haematopoietic stem cell), a bone marrow cell (e.g. a bone marrow mesenchymal cell or a bone marrow stromal sell), a central nervous system cell (e.g. a neuron such as a dopaminergic neuron or a motor neuron, a Purkinje cell, an astrocyte, an ependymal cell or an oligodendrocyte), a cancer cell such as a hepato-cellular carcinoma cell, a lung cell, or a retinal cell.

11. The method or use according to any of Paragraphs 3-10, wherein the cell is a cell line.

12. The method or use according to Paragraph 11 wherein the cell line is any of a HeLa cell, a Huh7 cell, a HMEC cell, a HEK293 cell, a HBEC cell, a HepG2 cell, a HT-29 cell, a Jurkat cell, a Saos-2 cell, a U2OS cell, a HUVEC cell or a fibroblast cell.

13. The method according to any one of Paragraphs 1 and 3-12 or the use according to any one of Paragraphs 2-12, wherein contacting the AAV with albumin involves incubating the AAV with albumin for at least 30 minutes.

14. The method according to any one of Paragraphs 1 and 3-13 or the use according to any one of Paragraphs 2-13, wherein contacting the AAV with albumin involves incubating the AAV with albumin at a temperature of from about 4°C to about room temperature, such as from about 4°C about 10°C, such as about 4°C.

15. The method according to any one of Paragraphs 1 and 3-14 or the use according to any one of Paragraphs 2-14, wherein the AAV is any one of AAV serotype 1 (AAV1), AAV2, AAV3, AAV4, AAV5, AAV6, AAV7, AAV8, AAV9, AAV10, AAV11, AAV12, AAV-DJ, AAV-DJ/8, AAV-Rh10, AAV-retro, AAV-PHP.B, AAV8-PHP.eB, AAV-PHP.S, preferably AAV2, AAV6 or AAV5.

16. The method according to any one of Paragraphs 1 and 3-15 or the use according to any one of Paragraphs 2-15, wherein the AAV does not contain an albumin-binding domain.

17. The method according to any one of Paragraphs 1 and 3-16 or the use according to any one of Paragraphs 2-16, wherein the anti-AAV neutralising antibody specifically binds to the AAV and inhibits an activity of the AAV.

18. The method according to any one of Paragraphs 1 and 3-17 or the use according to any one of Paragraphs 2-17, wherein the anti-AAV neutralising antibody is one that specifically binds to one or more of AAV serotype 1 (AAV1), AAV2, AAV3, AAV4, AAV5, AAV6, AAV7, AAV8, AAV9, AAV10, AAV11, AAV12, AAV-DJ, AAV-DJ/8, AAV-Rh10, AAV-retro, AAV-PHP.B, AAV8-PHP.eB, and AAV-PHP.S.

19. The method according to any one of Paragraphs 1 and 3-18 or the use according to any one of Paragraphs 2-18, wherein the anti-AAV neutralising antibody is one that specifically binds to AAV5, or is one that specifically binds to AAV3, AAV2 and AAV6, or is one that specifically binds to AAV2.

20. The method according to any one of Paragraphs 1 and 3-19 or the use according to any one of Paragraphs 2-19, wherein the albumin is sourced from a recombinant source or is sourced from serum.

21. The method according to any one of Paragraphs 1 and 3-20 or the use according to any one of Paragraphs 2-20, wherein the albumin is a wild-type albumin or a variant thereof, or ovalbumin or a variant thereof.

22. The method according to any one of Paragraphs 1 and 3-21 or the use according to any one of Paragraphs 2-21, wherein the albumin comprises an amino acid sequence of a mammalian albumin, optionally a human albumin, bovine albumin, dog albumin or mouse albumin.

23. The method according to any one of Paragraphs 1 and 3-22 or the use according to any one of Paragraphs 2-22, wherein the albumin is recombinant human albumin or human serum albumin.

24. The method according to any one of Paragraphs 1 and 3-23 or the use according to any one of Paragraphs 2-23, wherein the albumin is:

a) a yeast-derived albumin, optionally wherein the yeast is *Pichia* such as *Pichia pastoris, Saccharomyces* such as *Saccharomyces cerevisiae, Candida* such as *Candida utilis, Kluyveromyces* such as *Kluyveromyces lactis* or *Kluyveromyces marxianus, Hansenula polymorpha, Schizosaccharomyces pombe, Yarrowia lipolytica, Arxula adeninivorans,* or Zygosaccharomyces such as *Zygosaccharomyces bailii*; or
b) a plant-derived albumin, optionally a rice-derived albumin.

25. The method according to any one of Paragraphs 1 and 3-24 or the use according to any one of Paragraphs 2-24, wherein the albumin is a variant of a wild-type albumin, optionally wherein the variant albumin has at least 70%, 80%, 85%, 90%, 91%, 92%, 93%, 94%, 95%, 96%, 97%, 98%, or 99% sequence identity with the wild-type albumin.

26. The method according to any one of Paragraphs 1 and 3-25 or the use according to any one of Paragraphs 2-25, wherein the albumin is at a concentration of from about 1 mg/mL to about 400 mg/mL

27. The method according to any one of Paragraphs 1 and 3-26 or the use according to any one of Paragraphs 2-26, wherein the AAV is present at a multiplicity of infection (MOI) range of from about 0.1 to about $10^8$, such as from about $10^3$ to about $10^6$, such as about $10^5$.

28. The method according to any one of Paragraphs 1 and 3-27 or the use according to any one of Paragraphs 2-27, wherein the method or use is carried out *in vitro.*

29. A method of improving the efficacy of an adeno-associated virus (AAV) based therapy, the method comprising contacting an AAV with albumin prior to administering the AAV to a subject.

30. Use of albumin to improve the efficacy of an adeno-associated virus (AAV) based therapy, wherein the use involves contacting an AAV with albumin prior to administering the AAV to a subject.

31. A method of reducing the neutralisation of an adeno-associated virus (AAV) based therapy by an anti-AAV neutralising antibody, the method comprising contacting an AAV with albumin prior to administering the AAV to a subject.

32. Use of albumin to reduce the neutralisation of an adeno-associated virus (AAV) based therapy by an anti-AAV neutralising antibody, wherein the use involves contacting an AAV with albumin prior to administering the AAV to a subject.

33. The method according to Paragraph 29 or 31, further comprising administering the AAV to the subject.

34. The method according to any one of Paragraphs 29, 31 or 33, or the use according to Paragraph 30 or 32, wherein the method or use enables a higher therapeutic payload to be delivered to cells per AAV dose than the corresponding therapeutic payload delivered to cells per AAV dose when the AAV is not contacted with albumin prior to administering the AAV to a subject.

35. The method according to any one of Paragraphs 29, 31, 33 or 34, or the use according to any one of Paragraphs 30, 32 or 34, wherein the method or use enables a reduction in the effective dose of the AAV-based therapy.

36. The method according to any one of Paragraphs 29, 31 and 33-35, or the use according to any one of Paragraphs 30, 32, 34 or 35 wherein the method reduces adverse effects of an AAV-based therapy.

37. A method of treating a disease or condition with an AAV-based therapy, the method comprising (a) contacting an AAV with albumin prior to administering the AAV to a subject; and (b) administering the AAV to the subject.

38. A combination of an AAV and albumin for use in treating a disease or condition with an AAV-based therapy, wherein the AAV is contacted with albumin prior to administering the AAV to a subject.

39. Use of a combination of AAV and albumin in preparing a medicament for treating a disease or condition with an AAV-based therapy, wherein the AAV is contacted with albumin prior to administering the AAV to a subject.

40. The method according to any one of Paragraphs 29, 31 and 33-37, the combination for use according to Paragraph 38 or the use according to Paragraph 39, wherein the subject is one that has anti-AAV neutralising antibodies.

41. The method according to any one of Paragraphs 29, 31 and 33-37, the combination for use according to Paragraph 38 or the use according to Paragraph 39, wherein the subject is one that does not have anti-AAV neutralising antibodies.

42. The method according to any one of Paragraphs 29, 31, 33-37, 40 and 41, the combination for use according to any one of Paragraphs 38, 40 or 41, or the use according to any one of Paragraphs 30, 32, 34-36, and 39-41, wherein contacting the AAV with albumin is as defined in Paragraph 13 or 14.

43. The method according to any one of Paragraphs 29, 31, 33-37 and 40-42, the combination for use according to any one of Paragraphs 38 and 40-42 or the use according to any one of Paragraphs 30, 32, 34-36 and 39-42, wherein the AAV is as defined in any one of Paragraphs 15, 16 and 27.

44. The method according to any one of Paragraphs 31, 33-36 and 40-43, the combination for use according to any one of Paragraphs 40-43 or the use according to any one of Paragraphs 32, 34-36 and 40-43, wherein the anti-AAV neutralising antibody is as defined in any one of Paragraphs 17-19.

45. The method according to any one of Paragraphs 29, 31, 33-37 and 40-44, the combination for use according to any one of Paragraphs 38 and 40-44, or the use according to any one of Paragraphs 30, 32, 34-36 and 39-44 wherein the albumin is as defined in any one of Paragraphs 20-26.

**Claims**

1. A method of preserving the activity of an adeno-associated virus (AAV) in the presence of an anti-AAV neutralising antibody, the method comprising contacting the AAV with albumin.

2. Use of albumin to preserve the activity of an adeno-associated virus (AAV) in the presence of an anti-AAV neutralising antibody, wherein the use involves contacting the AAV with albumin.

3. The method according to Claim 1 or a use according to Claim 2, wherein the activity of the AAV is the ability of the AAV to transduce a cell, or the ability of the AAV to infect a cell.

4. The method or use according to Claim 3, wherein

(a) the level of transduction of the AAV into the cell when the AAV is contacted with albumin is higher than the level of transduction of the AAV into the cell when the AAV is not contacted with albumin; and/or
(b) the ability of the AAV to transduce a cell is assessed by a fluorescence-based method, bioluminescence, an enzyme-based method, DNA staining, polymerase chain reaction (PCR), enzyme-linked immunosorbent assay (ELISA) or western blotting; and/or
(c) the cell is a dividing cell or a non-dividing cell; and/or
(d) the cell is a eukaryotic cell; and/or
(e) the cell is an animal cell, optionally, wherein the animal cell is a mammalian cell, a fish cell, an insect cell, a reptile cell, an amphibian cell or an avian cell; and/or

(f) cell is any one of an epithelial cell (*e.g.* a nasal epithelial cell), a liver cell, a muscle cell, a heart cell, an immune cell, a pancreatic cell, an endothelial cell, a stem cell (a haematopoietic stem cell), a bone marrow cell (*e.g.* a bone marrow mesenchymal cell or a bone marrow stromal sell), a central nervous system cell (*e.g.* a neuron such as a dopaminergic neuron or a motor neuron, a Purkinje cell, an astrocyte, an ependymal cell or an oligodendrocyte), a cancer cell such as a hepato-cellular carcinoma cell, a lung cell, or a retinal cell; and/or the cell is a cell line, optionally wherein the cell line is any of a HeLa cell, a Huh7 cell, a HMEC cell, a HEK293 cell, a HBEC cell, a HepG2 cell, a HT-29 cell, a Jurkat cell, a Saos-2 cell, a U2OS cell, a HUVEC cell or a fibroblast cell.

5. The method according to any one of Claims 1, 3 and 4 or the use according to any one of Claims 2-4, wherein:

(a) contacting the AAV with albumin involves incubating the AAV with albumin for at least 30 minutes; and/or
(b) contacting the AAV with albumin involves incubating the AAV with albumin at a temperature of from about 4°C to about room temperature, such as from about 4°C about 10°C, such as about 4°C; and/or
(c) the AAV is any one of AAV serotype 1 (AAV1), AAV2, AAV3, AAV4, AAV5, AAV6, AAV7, AAV8, AAV9, AAV10, AAV11, AAV12, AAV-DJ, AAV-DJ/8, AAV-Rh10, AAV-retro, AAV-PHP.B, AAV8-PHP.eB, AAV-PHP.S, preferably AAV2, AAV6 or AAV5; and/or
(d) the AAV does not contain an albumin-binding domain; and/or
(e) the anti-AAV neutralising antibody specifically binds to the AAV and inhibits an activity of the AAV; and/or
(f) the anti-AAV neutralising antibody is one that specifically binds to one or more of AAV serotype 1 (AAV1), AAV2, AAV3, AAV4, AAV5, AAV6, AAV7, AAV8, AAV9, AAV10, AAV11, AAV12, AAV-DJ, AAV-DJ/8, AAV-Rh10, AAV-retro, AAV-PHP.B, AAV8-PHP.eB, and AAV-PHP.S; and/or
(g) the anti-AAV neutralising antibody is one that specifically binds to AAV5, or is one that specifically binds to AAV3, AAV2 and AAV6, or is one that specifically binds to AAV2; and/or
(h) the albumin is sourced from a recombinant source or is sourced from serum; and/or
(i) the albumin is a wild-type albumin or a variant thereof, or ovalbumin or a variant thereof; and/or
(j) the albumin comprises an amino acid sequence of a mammalian albumin, optionally a human albumin, bovine albumin, dog albumin or mouse albumin; and/or
(k) the albumin is recombinant human albumin or human serum albumin; and/or
(l) the albumin is:

la) a yeast-derived albumin, optionally wherein the yeast is *Pichia* such as *Pichia pastoris, Saccharomyces* such as *Saccharomyces cerevisiae, Candida* such as *Candida utilis, Kluyveromyces* such as *Kluyveromyces lactis* or *Kluyveromyces marxianus, Hansenula polymorpha, Schizosaccharomyces pombe, Yarrowia lipolytica, Arxula adeninivorans,* or Zygosaccharomyces such as *Zygosaccharomyces bailii*; or
lb) a plant-derived albumin, optionally a rice-derived albumin; and/or (m) the albumin is a variant of a wild-type albumin, optionally wherein the variant albumin has at least 70%, 80%, 85%, 90%, 91%, 92%, 93%, 94%, 95%, 96%, 97%, 98%, or 99% sequence identity with the wild-type albumin; and/or (n) the albumin is at a concentration of from about 1 mg/mL to about 400 mg/mL; and/or

(o) the AAV is present at a multiplicity of infection (MOI) range of from about 0.1 to about $10^8$, such as from about $10^3$ to about $10^6$, such as about $10^5$; and/or
(p) the method or use is carried out *in vitro.*

6. A method of improving the efficacy of an adeno-associated virus (AAV) based therapy, the method comprising contacting an AAV with albumin prior to administering the AAV to a subject.

7. Use of albumin to improve the efficacy of an adeno-associated virus (AAV) based therapy, wherein the use involves contacting an AAV with albumin prior to administering the AAV to a subject.

8. A method of reducing the neutralisation of an adeno-associated virus (AAV) based therapy by an anti-AAV neutralising antibody, the method comprising contacting an AAV with albumin prior to administering the AAV to a subject.

9. Use of albumin to reduce the neutralisation of an adeno-associated virus (AAV) based therapy by an anti-AAV neutralising antibody, wherein the use involves contacting an AAV with albumin prior to administering the AAV to a subject.

10. The method according to Claim 6 or 8, further comprising administering the AAV to the subject.

11. The method according to any one of Claims 6, 8 or 10, or the use according to Claim 7 or 9, wherein

(a) the method or use enables a higher therapeutic payload to be delivered to cells per AAV dose than the corresponding therapeutic payload delivered to cells per AAV dose when the AAV is not contacted with albumin prior to administering the AAV to a subject; and/or
(b) the method or use enables a reduction in the effective dose of the AAV-based therapy; and/or
(c) the method reduces adverse effects of an AAV-based therapy.

12. A method of treating a disease or condition with an AAV-based therapy, the method comprising (a) contacting an AAV with albumin prior to administering the AAV to a subject; and (b) administering the AAV to the subject.

13. A combination of an AAV and albumin for use in treating a disease or condition with an AAV-based therapy, wherein the AAV is contacted with albumin prior to administering the AAV to a subject.

14. Use of a combination of AAV and albumin in preparing a medicament for treating a disease or condition with an AAV-based therapy, wherein the AAV is contacted with albumin prior to administering the AAV to a subject.

15. The method according to any one of Claims 6, 8 and 10-12, the combination for use according to Claim 13 or the use according to Claim 14, wherein

(a) the subject is one that has anti-AAV neutralising antibodies; or
(b) the subject is one that does not have anti-AAV neutralising antibodies.

16. The method according to any one of Claims 6, 8, 10-12 and 15, the combination for use according to Claim 13 or 15, or the use according to any one of Claims 7, 9, 11 and 14-15, wherein:

(a) contacting the AAV with albumin is as defined in Claim 5(a) or 5(b); and/or
(b) the AAV is as defined in any one of Claims 5(c), 5(d) and 5(o); and/or
(c) the anti-AAV neutralising antibody is as defined in any one of Claims 5(e)-(g); and/or
(d) the albumin is as defined in any one of Claims 5(h)-(n).

# Figure 1

# Figure 2

# Figure 3

| | | | | AAV+buffer+Ab | | | AAV+albumin+Ab | | | | |
|---|---|---|---|---|---|---|---|---|---|---|---|
| - | - | - | - | - | - | - | - | - | - | - | - |
| - | Cells only | AAV+buffer only | AAV+albumin only | 1/30 Ab | 1/30 Ab | 1/30 Ab | 1/30 Ab | 1/30 Ab | 1/30 Ab | - | - |
| - | Cells only | AAV+buffer only | AAV+albumin only | 1/90 Ab | 1/90 Ab | 1/90 Ab | 1/90 Ab | 1/90 Ab | 1/90 Ab | - | - |
| - | Cells only | AAV+buffer only | AAV+albumin only | 1/270 Ab | 1/270 Ab | 1/270 Ab | 1/270 Ab | 1/270 Ab | 1/270 Ab | - | - |
| - | - | - | - | 1/810 Ab | 1/810 Ab | 1/810 Ab | 1/810 Ab | 1/810 Ab | 1/810 Ab | - | - |
| - | - | - | - | 1/2430 Ab | 1/2430 Ab | 1/2430 Ab | 1/2430 Ab | 1/2430 Ab | 1/2430 Ab | - | - |
| - | - | - | - | 1/7290 Ab | 1/7290 Ab | 1/7290 Ab | 1/7290 Ab | 1/7290 Ab | 1/7290 Ab | - | - |
| - | - | - | - | - | - | - | - | - | - | - | - |

Europäisches
Patentamt

European
Patent Office

Office européen
des brevets

**EUROPEAN SEARCH REPORT**

**Application Number**

**EP 24 19 7402**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WANG M ET AL: "Direct interaction of human serum proteins with AAV virions to enhance AAV transduction: immediate impact on clinical applications", GENE THERAPY, NATURE PUBLISHING GROUP, LONDON, GB, vol. 24, no. 1, 11 November 2016 (2016-11-11), pages 49-59, XP037648981, ISSN: 0969-7128, DOI: 10.1038/GT.2016.75 [retrieved on 2016-11-11] * page 5 col 1 par 2 and col 2 par 2, page 7 col 1 par 2; figures 7b,8b * | 1-16 | INV. C12N7/00 C12N15/86 |
| X | EP 4 303 225 A2 (UNIV NORTH CAROLINA CHAPEL HILL [US]) 10 January 2024 (2024-01-10) * paragraphs [0354], [0358] - [0360]; figure 8 * | 1-16 | |
| X | WRIGHT J FRASER: "Coating of AAV Vectors with Human Albumin Blocks Antibody Binding and Enables Transduction of Human Hepatocytes in the Presence of Neutralizing Antibodies", BLOOD, W.B. SAUNDERS, vol. 112, no. 11, 16 November 2008 (2008-11-16), page 3542, XP086682521, ISSN: 0006-4971, DOI: 10.1182/BLOOD.V112.11.3542.3542 * the whole document * | 1-5,7,11 | **TECHNICAL FIELDS SEARCHED (IPC)** C12N C07K |

-/--

The present search report has been drawn up for all claims

| Place of search | Date of completion of the search | Examiner |
|---|---|---|
| Munich | 5 February 2025 | Dogrammatzis, C |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 2

Europäisches
Patentamt

European
Patent Office

Office européen
des brevets

# EUROPEAN SEARCH REPORT

**Application Number**

EP 24 19 7402

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | JIN QUAN ET AL: "An engineered serum albumin-binding AAV9 capsid achieves improved liver transduction after intravenous delivery in mice", GENE THERAPY, NATURE PUBLISHING GROUP, LONDON, GB, vol. 27, no. 5, 9 December 2019 (2019-12-09), pages 237-244, XP037147905, ISSN: 0969-7128, DOI: 10.1038/S41434-019-0107-2 [retrieved on 2019-12-09] * page 5 col 1 par 1 * | 4 | |
| A | WRIGHT J. FRASER: "Re-administration of AAV vectors by masking with host albumin: A Goldilocks hypothesis", MOLECULAR THERAPY, vol. 31, no. 7, 1 July 2023 (2023-07-01), pages 1870-1873, XP093246500, ISSN: 1525-0016, DOI: 10.1016/j.ymthe.2023.06.009 * page 1 col 2 * | 4 | |
| | | | **TECHNICAL FIELDS SEARCHED (IPC)** |

The present search report has been drawn up for all claims

| Place of search | Date of completion of the search | Examiner |
|---|---|---|
| Munich | 5 February 2025 | Dogrammatzis, C |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

 

& : member of the same patent family, corresponding document

EPO FORM 1503 03.82 (P04C01)

page 2 of 2

## ANNEX TO THE EUROPEAN SEARCH REPORT
## ON EUROPEAN PATENT APPLICATION NO.

EP 24 19 7402

This annex lists the patent family members relating to the patent documents cited in  the above-mentioned European search report.
The members are as contained in the European Patent Office EDP file on
The European Patent Office is in no way liable for these particulars which are merely given for the purpose of information.

05-02-2025

| Patent document cited in search report | | Publication date | Patent family member(s) | | Publication date |
|---|---|---|---|---|---|
| EP 4303225 | A2 | 10-01-2024 | AU | 2018234695 A1 | 12-09-2019 |
| | | | AU | 2024227303 A1 | 31-10-2024 |
| | | | CA | 3054711 A1 | 20-09-2018 |
| | | | CN | 110770346 A | 07-02-2020 |
| | | | CN | 117801075 A | 02-04-2024 |
| | | | EP | 3610023 A1 | 19-02-2020 |
| | | | EP | 4303225 A2 | 10-01-2024 |
| | | | ES | 2966692 T3 | 23-04-2024 |
| | | | IL | 268891 A | 31-10-2019 |
| | | | IL | 312266 A | 01-06-2024 |
| | | | IL | 316259 A | 01-12-2024 |
| | | | JP | 7132934 B2 | 07-09-2022 |
| | | | JP | 2020511127 A | 16-04-2020 |
| | | | WO | 2018170310 A1 | 20-09-2018 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2013006675 A
- EP 624195 A
- WO 2001079271 A
- WO 2003059934 A
- WO 2003060071 A
- WO 2011051489 A
- WO 2011124718 A
- EP 11164955 A
- WO 2012059486 A
- WO 2012150319 A
- WO 2014072481 A
- WO 2013135896 A
- WO 2015036579 A
- WO 2010092135 A
- WO 2013075066 A
- WO 2014179657 A
- WO 2009126920 A
- WO 2010059315 A
- WO 2011103076 A
- WO 2012112188 A
- WO 2015063611 A
- WO 2017029407 A
- WO 06066595 A
- EP 73646 A

**Non-patent literature cited in the description**

- **NIELSEN et al.** *Protein Engineering*, 1997, vol. 10 (1), 1-6
- **PETERSEN et al.** *Nat Methods*, 2011, vol. 8 (10), 785-786
- **KRAGH-HANSEN et al.** *Biol Pharm Bull*, 2002, vol. 25 (6), 695-704
- **NEEDLEMAN** ; **WUNSCH**. *J Mol Biol*, 1970, vol. 48 (3), 443-453
- **RICE et al.** EMBOSS: The European Molecular Biology Open Software Suite. *Trends Genet*, 2000, vol. 16 (6), 276-277
- **FLEER**. *Bio/technology*, 1991, vol. 9, 968-975
- **KOBAYASHI**. *Therapeutic Apheresis*, 1998, vol. 2, 257-262
- **SLEEP**. *Bio/technology*, 1990, vol. 8, 42-46
- **BARASH**. *Transgenic Research*, 1993, vol. 2, 266-276
- **SIJMONS**. *Biotechnology*, 1990, vol. 8, 217
- **FARRAN**. *Transgenic Research*, 2002, vol. 11, 337-346
- **PANDJAITAB**. *J. Allergy Clin. Immunol.*, 2000, vol. 105, 279-285
- **CELIK** ; **CALIK**. *Biotechnology Advances*, 2012, vol. 30 (5), 1108-1118
- Established and Upcoming Yeast Expression Systems. **GUNDUZ ERGUN et al.** Recombinant Protein Production in Yeast. Humana, 2019, vol. 1923, 1-74
- **AMIDON et al.** *Pharm Res.*, 1995, vol. 12 (3), 413-420
- **CALCEDO, R.** ; **WILSON, J. M.** Humoral Immune Response to AAV. *Frontiers in Immunology*, 2013, vol. 4, https://doi.org/10.3389/fimmu.2013.00341
- Evading and overcoming AAV neutralization in gene therapy. **EARLEY, J.** ; **PILETSKA, E.** ; **RONZITTI, G.** ; **PILETSKY, S.** Trends in Biotechnology. Elsevier Ltd., 2023, vol. 41, 836-845
- **FRANCIS, G. L.** Albumin and mammalian cell culture: Implications for biotechnology applications. *Cytotechnology*, 2010, vol. 62 (1), 1-16, https://doi.org/10.1007/s10616-010-9263-3
- Overcoming the Challenges Imposed by Humoral Immunity to AAV Vectors to Achieve Safe and Efficient Gene Transfer in Seropositive Patients. **GROSS, D. A.** ; **TEDESCO, N.** ; **LEBORGNE, C.** ; **RONZITTI, G.** Frontiers in Immunology. Frontiers Media S.A, 2022, vol. 13
- Various AAV Serotypes and Their Applications in Gene Therapy: An Overview. **ISSA, S. S.** ; **SHAIMARDANOVA, A. A.** ; **SOLOVYEVA, V. V.** ; **RIZVANOV, A. A.** Cells. MDPI, 2023, vol. 12
- **LAUGHLIN, C. A.** ; **CARDELLICHIO, C. B.** ; **COON, H. C.** Latent Infection of KB Cells with Adeno-Associated Virus Type 2. *JOURNAL OF VIROLOGY*, 1986, vol. 60 (2)
- Albumin corona on nanoparticles-a strategic approach in drug delivery. **MARIAM, J.** ; **SIVAKAMI, S.** ; **DONGRE, P. M.** Drug Delivery. Taylor and Francis Ltd, 2016, vol. 23, 2668-2676

- **MATSUSHITA, T.** ; **ELLIGER, S.** ; **ELLIGER, C.** ; **PODSAKOFF, G.** ; **VILLARREAL, L.** ; **KURTZMAN, G. J.** ; **IWAKI, Y.** ; **COLOSI, P.** Adeno-associated virus vectors can be efficiently produced without helper virus. *Gene Therapy*, 1998, vol. 5, http://www.stockton-press.co.uk/gt

- Testing preexisting antibodies prior to AAV gene transfer therapy: rationale, lessons and future considerations. **MENDELL, J. R.** ; **CONNOLLY, A. M.** ; **LEHMAN, K. J.** ; **GRIFFIN, D. A.** ; **KHAN, S. Z.** ; **DHARIA, S. D.** ; **QUINTANA-GALLARDO, L.** ; **RODINO-KLAPAC, L. R.** Molecular Therapy Methods and Clinical Development. Cell Press, 2022, vol. 25, 74-83

- **SAND, K. M. K.** ; **BERN, M.** ; **NILSEN, J.** ; **DALHUS, B.** ; **GUNNARSEN, K. S.** ; **CAMERON, J.** ; **GREVYS, A.** ; **BUNTING, K.** ; **SANDLIE, I.** ; **ANDERSEN, J. T.** Interaction with Both Domain I and III of Albumin Is Required for Optimal pH-dependent Binding to the Neonatal Fc Receptor (FcRn) *. *Journal of Biological Chemistry*, 2014, vol. 289 (50), 34583-34594, https://doi.org/ 10.1074/j bc. M 114.587675

- **SARKAR, S. N.** ; **CORBIN, D.** ; **SIMPKINS, J. W.** Brain-Wide Transgene Expression in Mice by Systemic Injection of Genetically Engineered Exosomes: CAP-Exosomes. *Pharmaceuticals*, 2024, vol. 17 (3), https://doi.org/10.3390/ph17030270

- Adeno-associated virus as a delivery vector for gene therapy of human diseases. **WANG, J. H.** ; **GESSLER, D. J.** ; **ZHAN, W.** ; **GALLAGHER, T. L.** ; **GAO, G.** Signal Transduction and Targeted Therapy. Springer Nature, 2024, vol. 9

- **WANG, M.** ; **SUN, J.** ; **CROSBY, A.** ; **WOODARD, K.** ; **HIRSCH, M. L.** ; **SAMULSKI, R. J.** ; **LI, C.** Direct interaction of human serum proteins with AAV virions to enhance AAV transduction: Immediate impact on clinical applications. *Gene Therapy*, 2017, vol. 24 (1), 49-59, https://doi.org/10.1038/gt.2016.75